# EUROPEAN PATENT APPLICATION

(11) **EP 2 789 610 A1**
(43) Date of publication of application: **15.10.2014**
(21) Application number: 13163019.6
(22) Date of filing: 10.04.2013
(51) Int. Cl.: C07D 405/06, C07D 405/12

(54) **Purification of Posaconazole Intermediates**

(71) Applicant: SANDOZ AG, 4056 Basel (CH)
(72) Inventor: The designation of the inventor has not yet been filed
(74) Representative: Kunic Tesovic, Barbara

(57) **Abstract**

The present invention relates to a process for the purification of chiral compounds, in particular to the purification of a chiral compound of formula (XI) which may be used as intermediate for the preparation of antifungal agents, preferably posaconazole.

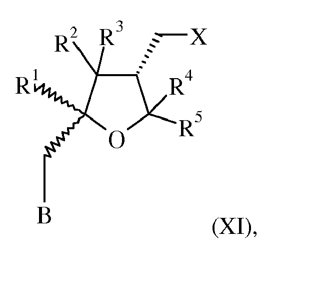

## Description

The present invention relates to the purification of chiral compounds, in particular to the purification of a chiral compound which may be used as intermediate for the preparation of antifungal agents, preferably posaconazole, and to the purified chiral compounds as such. Further, the present invention relates to these intermediates and their use for the preparation of antifungal agents, preferably posaconazole.

### Background prior art

Posaconazole (CAS Registry Number 171228-49-2; CAS Name: 2,5-anhydro-1,3,4-trideoxy-2-C-(2,4-difluorophenyl)-4-[[4-[4-[4-[1-[(1S,2S)-1-ethyl-2-hydroxypropyl]-1,5-dihydro-5-oxo-4H-1,2,4-triazol-4-yl]phenyl]-1-piperazinyl]phenoxy]methyl]-1-(1H-1,2,4-triazol-1-yl)-D-threo-pentitol) is a triazole antifungal drug represented by the structure:

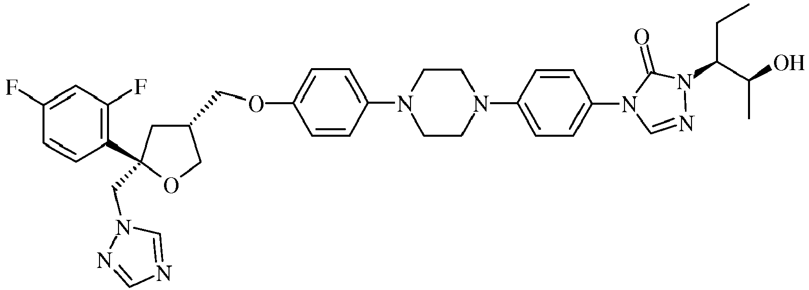

Posaconazole is used, for example, to prevent and/or treat invasive fungal infections caused by *Candida* species, *Mucor* species, *Aspergillus* species, *Fusarium* species, or *Coccidioides* species in immunocompromised patients and/or in patients where the disease is refractory to other antifungal agents such as amphothericin B, fluconazole, or itraconazole, and/or in patients who do not tolerate these antifungal agents.

One of the important intermediates for the preparation of posaconazole is the compound of formula (IIa) 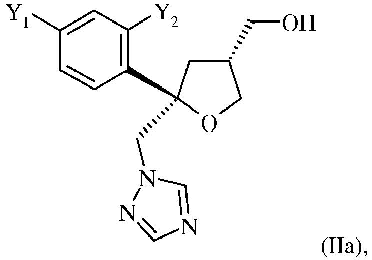 wherein both residues Y₁ and Y₂ are F. In a conceivable approach concerning the synthesis of the compound of formula (IIa), the quaternary stereocenter is generated by a iodocyclization, giving the required tetrahydrofuran as a diastereomeric mixture. In this mixture, the molar ratio of the *cis*-isomer relative to the *trans*-isomer (*cis*:*trans*) is in the range of from 85:15 to 95:5, typically about 9:1. However, only the *cis*-isomer is the desired starting material for the synthesis of posaconazole. Consequently, an enrichment of this desired *cis*-isomer or a suitable salt thereof is necessary.

According to the prior art, a diastereomeric mixture comprising, in a solvent, the *cis-*isomer of formula (IIa) and the trans-isomer of formula (IIIa) 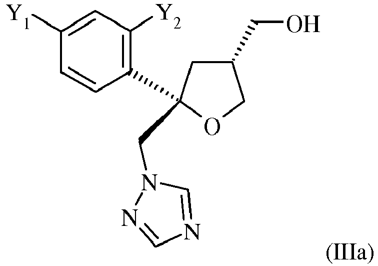 was converted, in a first step, to a diastereomeric mixture of the respective tosylates of compounds (IIa) and (IIIa) exhibiting the same *cis:trans* ratio as the starting mixture, i.e. typically the 9:1 ratio. From this diastereomeric mixture, the *cis*-isomer had to be separated in a second step via tedious gradient column chromatography on silica gel using large volumes of a mixture of heptane and ethyl acetate. In this context, reference is made to US 5,403,937, EP 0 736 030 A1, and WO 95/17407. However, column chromatography in general and the above-discussed column chromatography processes in particular are not suitable for industrial-scale processes.

Therefore, it was an object of the present invention to provide a new and advantageous process for the separation of the *cis*-isomer of a furan derivative comprising at least one hydroxyl group which can be halogenated, and at least one basic substituent from the respective *trans*-isomer. In particular, it was an object of the present invention to provide a new process for the separation of the *cis*-isomer of formula (IIa), from the *trans-*isomer of formula (IIIa).

Looking for such a new and advantageous process, it was found that most likely, making use of the known diastereomeric mixture of the tosylate of such furan derivatives, in particular compounds (IIa) and (IIIa), is not a promising starting point.

Therefore, it was an object of the present invention to provide a new intermediate containing suitable derivatives of the compounds of formula (IIa) and (IIIa).

### Summary of the invention

Surprisingly, it was found that the above-mentioned separation of the *cis*-isomer of a tetrahydrofuran derivative comprising at least one hydroxyl group which can be halogenated and at least one basic substituent, from the respective *trans*-isomer, in particular of the compound of formula (IIa), from the compound of formula (IIIa), can be considerably simplified if, starting from the diastereomeric mixture containing both isomers, in particular the compounds of formula (IIa) and (IIIa), a specific salt is prepared. This specific salt was found to be the HCl salt of the reaction product of a furan derivative comprising at least one hydroxyl group which can be halogenated and at least one basic substituent and a chlorination agent, in particular thionyl chloride. In particular, it was found to be the HCl salt of the reaction product of the compound of formula (Ia) 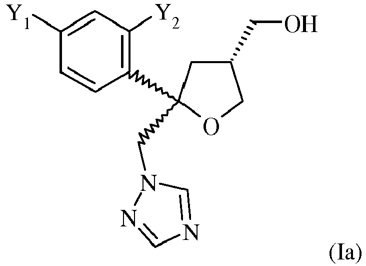 and a chlorination agent, in particular thionyl chloride.

Further, it has been found that compounds of formula (XI) 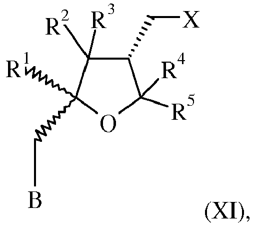 or their corresponding HX salts, in particular of formula (XIa) 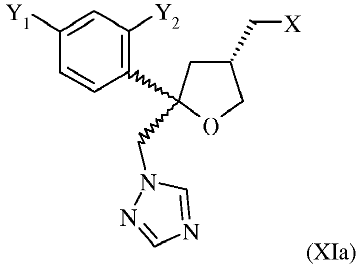 or their corresponding HX salts, wherein X is Cl, Br, or I, preferably Cl, are useful intermediates in the preparation of certain antifungal agents, preferably posaconazole.

Therefore, the present invention relates to a compound of formula (XI) 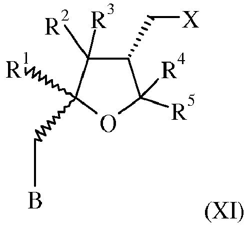 or its corresponding HX salt, wherein
- R¹: is a linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂- aryl alkyl residue, or a substituted or unsubstituted C₅-C₂₂-heteroaryl residue;
- R², R³, R⁴, R⁵: are, independently of each other, hydrogen, a linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂-aryl alkyl residue, a substituted or unsubstituted C₅-C₂₂-heteroaryl residue;
- B: is a basic substituent;
- X: is Cl, Br, or I;
said compound of formula (XI) or its corresponding HX salt containing the *cis*-isomer of formula (XII) 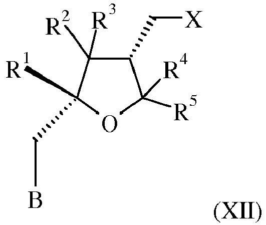 or its corresponding HX salt and the *trans-isomer* of formula (XIII) 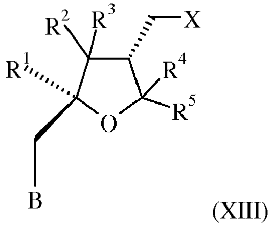 or its corresponding HX salt. Preferably, the compound of formula (XI) or its corresponding HX salt is at least partially crystalline, particularly preferably crystalline.

Further, the present invention relates to a process comprising
(1) providing a compound of formula (I) 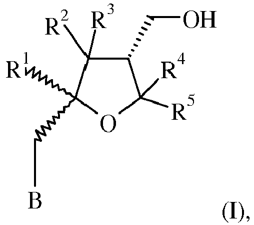 wherein
   - R¹: is a linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂- aryl alkyl residue, or a substituted or unsubstituted C₅-C₂₂-heteroaryl residue;
   - R², R³, R⁴, R⁵: are, independently of each other, a hydrogen, linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂-aryl alkyl residue, or a substituted or unsubstituted C₅-C₂₂-heteroaryl residue;
   - B: is a basic substituent;
   said compound of formula (I) containing the *cis*-isomer of formula (II) 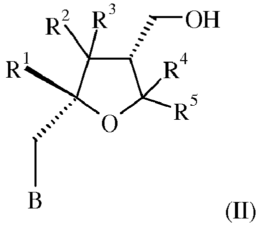 and the trans-isomer of formula (III) 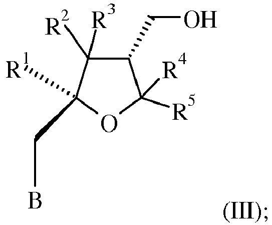
(2) treating the compound of formula (I) provided in (1) with at least one halogenation agent to obtain a compound of formula (XI) 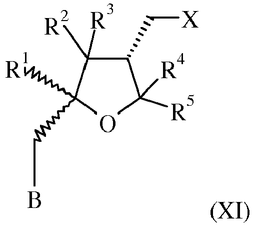 or its corresponding HX salt, containing the *cis*-isomer of formula (XII) 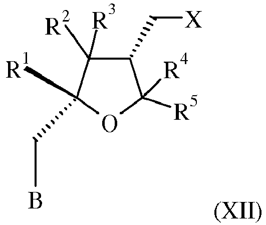 or its corresponding HX salt and the *trans-isomer* of formula (XIII) 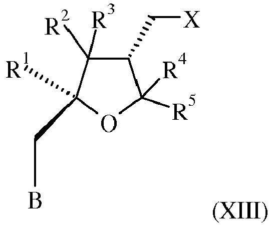 or its corresponding HX salt;
   wherein X is Cl, Br, or 1.

Yet further, the present invention relates to a compound of formula (XI) or its HX salt, obtainable or obtained by said process.

Still further, the present invention relates to the use of a compound of formula (XIa) 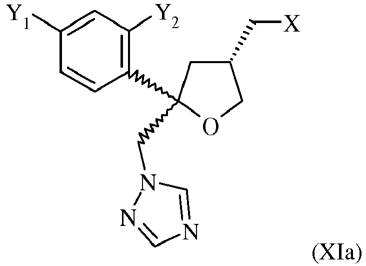 or its corresponding HX salt, wherein Y₁ and Y₂ are independently F or Cl, preferably F, and X is Cl, Br or I, preferably Cl, containing at least 97 %, preferably at least 98 % and more preferably at least 99 % of the *cis*-isomer of formula (XIIa) 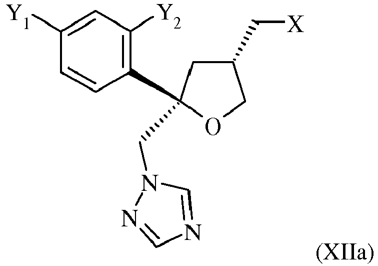 or its corresponding HX salt and at most 3 %, preferably at most 2 % and more preferably at most 1 % of the trans-isomer of formula (XIIIa) 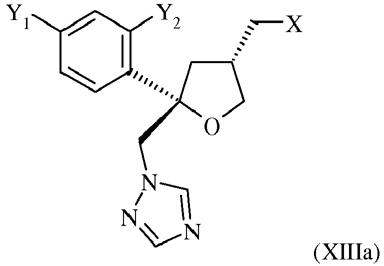 or its corresponding HX salt, for the preparation of an antifungal agent or a pharmaceutically acceptable salt thereof, preferably, wherein both Y₁ and Y₂ are F, of a compound of formula (X) 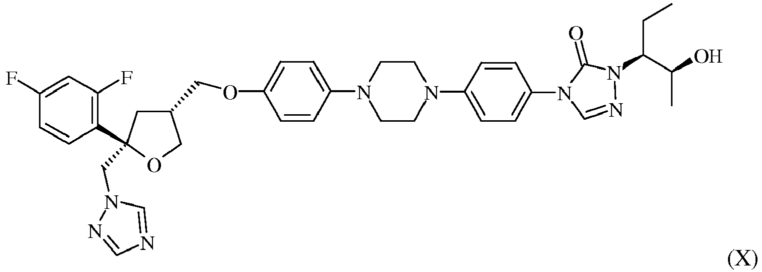 or a pharmaceutically acceptable salt thereof. Preferably, the compound of formula (XIa) or its corresponding HX salt is at least partially crystalline, particularly preferably crystalline.

Also, the present invention relates to a process for the preparation of an antifungal agent or a pharmaceutically acceptable salt thereof, preferably of a compound of formula (X) 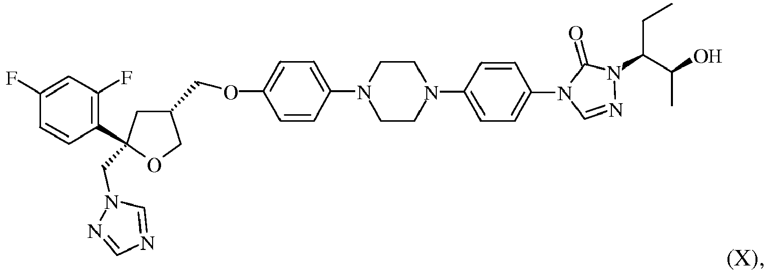 or a pharmaceutically acceptable salt thereof, wherein a compound of formula (XIa) 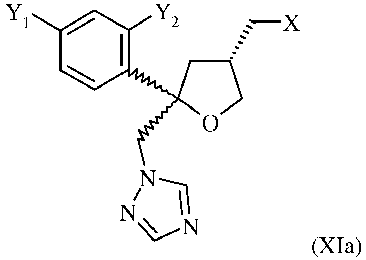 or its corresponding HX salt, wherein Y₁ and Y₂ are independently F or Cl, preferably F, and X is Cl, Br or I, preferably Cl, containing at least 97 %, preferably at least 98 % and more preferably at least 99 % of the *cis*-isomer of formula (XIIa) 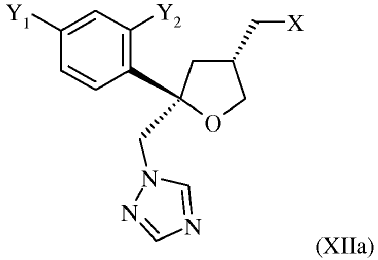 or its corresponding HX salt and at most 3 %, preferably at most 2 % and more preferably at most 1 % of the trans-isomer of formula (XIIIa) 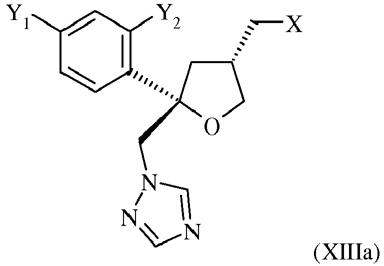 or its corresponding HX salt, is used as a starting material. Preferably, the compound of formula (XIa) or its corresponding HX salt is at least partially crystalline, particularly preferably crystalline.

### Short description of the Figures

- Fig. 1: shows the X-ray powder diffraction pattern of the polymorphic form I of the compound of formula (Va) as prepared according to Example 2.
- Fig. 2: shows the X-ray powder diffraction pattern of the polymorphic form II of the compound of formula (Va) as prepared according to Example 2.
- Fig. 3: shows the X-ray powder diffraction pattern of the mixture of the polymorphic form I and the polymorphic form II of the compound of formula (Va) as prepared according to Example 2.
- Fig. 4: shows the X-ray powder diffraction pattern of the compound of formula (XIa) as prepared according to Example 8.

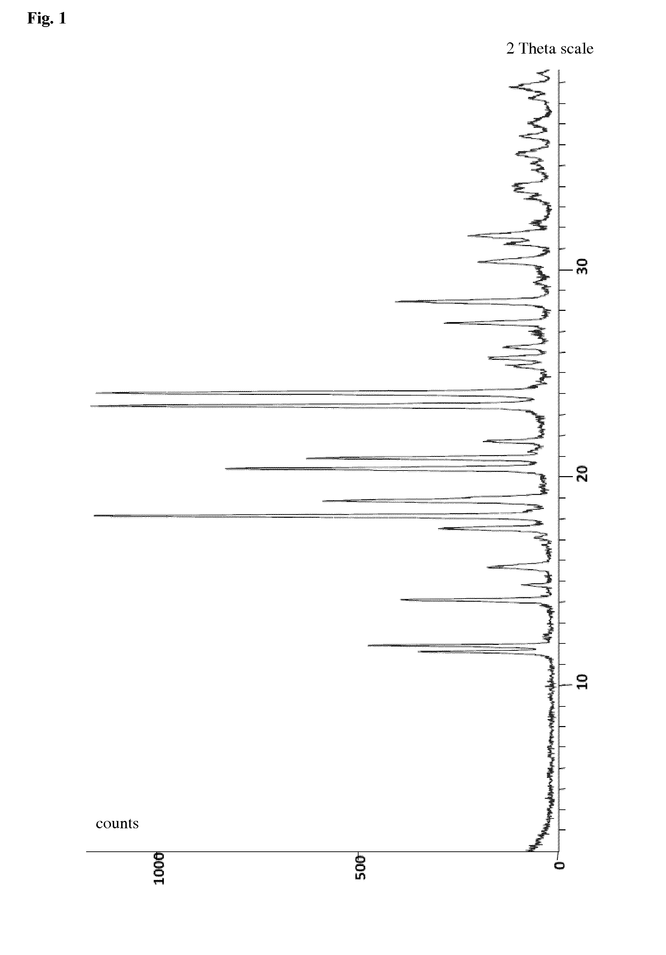

In Figures 1 to 4, intensity (counts) is presented on the y-axis, while the position (2 theta values in degrees, 2-Theta-Scale) is presented on the x-axis.

### Detailed description

According to step (1) of the process according to the present invention a compound of formula (I) 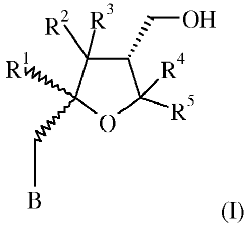 is provided.

### Residue R¹

Generally, R¹ is a linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂- aryl alkyl residue, or a substituted or unsubstituted C₅-C₂₂-heteroaryl residue. Suitable linear or branched, substituted or unsubstituted C₁-C₁₂-alkyl residues, i.e. alkyl residues having from 1 to 12 carbon atoms, include, but are not restricted to, methyl, ethyl, propyl such as n-propyl, iso-propyl, and butyl such as n-butyl, iso-butyl and tert-butyl.

Suitable substituted or unsubstituted C₅-C₂₂-aryl residues, preferably C₅-C₁₂-aryl residues, include, but are not restricted to, phenyl, biphenyl, naphthyl, in particular phenyl. Preferred substituents are, for example, C₁-C₁₂-alkyl residues as defined above and/or halogens such as F, Cl, Br and/or I, particularly preferably F and/or Cl. Preferably, R¹ is a substituted, preferably disubstituted phenyl, in particular disubstituted by F and/or Cl. More preferably, R¹ is a 2,4-disubstituted phenyl, particularly preferably of the following formula 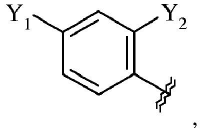 wherein Y₁ and Y₂ are independently F or Cl, preferably F, wherein the double sinuous line indicates the bond via which the disubstituted phenyl is attached to the furan in formula (I).

Suitable linear or branched, substituted or unsubstituted C₆-C₂₂-alkyl aryl residues, preferably C₆-C₁₃-alkyl aryl residues, include, but are not restricted to, benzyl, alkyl phenyl and methyl naphthyl.

Suitable linear or branched, substituted or unsubstituted C₆-C₂₂- aryl alkyl residues, preferably C₆-C₁₃-aryl alkyl residues, include, but are not restricted to, toluyl, xylyl and mesityl.

Suitable substituted or unsubstituted C₅-C₂₂-heteroaryl residues, preferably C₅-C₁₃-heteroaryl residues, include, but are not restricted to, residues which are derived from the aryl residues as defined above by substitution of at least one carbon-ring-atom with a heteroatom selected from the group consisting of N, O, P and S. Examples of suitable heterocycles forming said heteroaryl residues include pyrrole, imidazole, triazole, tetrazole, thiazole, oxazole, pyridine, piperidine, pyrazin, pyrimidine, pyridazin, piperazine, morpholine, azepine, indole, benzimidazole, purine, chinoline, isochinoline, phenothiazine and pyrazine.

### Residues R², R³, R⁴, R⁵

Generally, R², R³, R⁴, R⁵ are, independently of each other, hydrogen, linear or branched, substituted or unsubstituted C₁-C₁₂-alkyl residues, substituted or unsubstituted C₅-C₂₂-aryl residues, linear or branched, substituted or unsubstituted C₆-C₂₂-alkyl aryl residues, linear or branched, substituted or unsubstituted C₆-C₂₂-aryl alkyl residues, or substituted or unsubstituted C₅-C₂₂-heteroaryl residues.

Suitable branched, substituted or unsubstituted C₁-C₁₂-alkyl residues include, but are not limited to, methyl, ethyl, propyl such as n-propyl, iso-propyl, and butyl such as n-butyl, iso-butyl or tert-butyl.

Suitable substituted or unsubstituted C₅-C₂₂-aryl residues, preferably C₅-C₁₂-aryl residues, include, but are not limited to, phenyl, biphenyl and naphthyl, in particular phenyl. If substituted, preferred substituents are selected among C₁-C₁₂-alkyl residues as mentioned above and/or halogens such as F, Cl, Br and/or I, particularly preferably F and/or Cl.

Suitable linear or branched, substituted or unsubstituted C₆-C₂₂-alkyl aryl residues, preferably C₆-C₁₃-alkyl aryl residues, include, but are not limited to, benzyl, any alkyl phenyl and methylnaphthyl.

Suitable linear or branched, substituted or unsubstituted C₆-C₂₂- aryl alkyl residues, preferably C₆-C₁₃- aryl alkyl residues, include, but are not limited to, toluyl, xylyl and mesityl.

Suitable substituted or unsubstituted C₅-C₂₂-heteroaryl residues, preferably C₅-C₁₃-heteroaryl residues, include, but are not restricted to, residues which are derived from the aryl residues as defined above by substitution of at least one carbon-ring-atom by a heteroatom selected from N, O, P or S. Examples of suitable heterocycles forming said heteroaryl residues include pyrrole, imidazole, triazole, tetrazole, thiazole, oxazole, pyridine, piperidine, pyrazin, pyrimidine, pyridazin, piperazine, morpholine, azepine, indole, benzimidazole, purine, chinoline, isochinoline and phenothiazine.

In a particularly preferred embodiment of the present invention, R², R³, R⁴ and R⁵ are each hydrogen.

### Basic substituent B

In the context of the present invention, the term "basic substituent" refers to a substituent having a basic character, in that it can be converted into a salt by reaction with a strong acid such as HCl, H₂SO₄, HNO₃, HBr, H₃PO₄ or mixtures of two or more of these acids.

Suitable basic substituents include, but are not restricted to, substituents comprising one or more nitrogen atoms in a ring system or one or more nitrogen atoms as part of a carbon-chain.

Preferably, the basic substituent B is a heterocyclic residue having from 4 to 22 ring atoms, preferably from 4 to 12 ring atoms, particularly preferably 5 or 6 ring atoms, including the heteroatom(s) in each case, wherein the residue comprises at least one heteroatom. Preferably, at least one heteroatom of the residue is N. Optionally, the residue additionally contains at least one heteroatom which is selected from O, P and/or S. Examples of suitable heterocycles forming said heterocyclic residues include pyrrole, imidazole, triazole, tetrazole, thiazole, oxazole, pyridine, piperidine, pyrazin, pyrimidine, pyridazin, piperazine, morpholine, azepine, indole, benzimidazole, purine, chinoline, isochinoline, phenothiazine and pyrazine.

A particularly preferred basic substituent according to the present invention is a triazole residue of the following formula 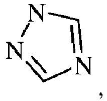 which can be connected, as shown, for example, in formula (I), via the CH₂-moiety to the furan ring system via any possible position. In a preferred embodiment, the triazole residue is connected in 1-position via the CH₂-moiety to the furan ring system. Therefore, according to a preferred embodiment of the present invention, the basic substituent B is 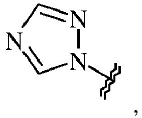 wherein the double sinuous line indicates the bond via which the triazole residue is connected via the CH₂-moiety to the furan in formula (I).

According to the present invention, the compound of formula (I) contains the *cis*-isomer of formula (II) and the trans-isomer of formula (III)

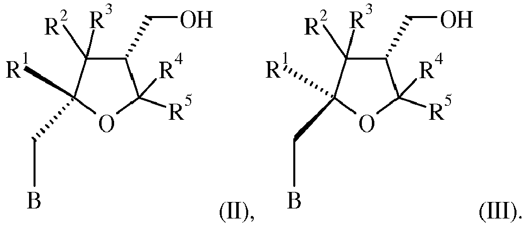

Generally, any conceivable combination of possible and preferred compounds according to general formulae (I), (II) and (III) is within the scope of the present invention. Most preferably, the present invention relates to above-defined process, wherein in step (1), a compound (I) is provided which is a compound of formula (Ia) 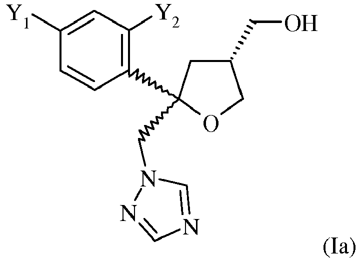 wherein Y₁ and Y₂ are independently F or Cl, preferably F, said compound of formula (Ia) containing the *cis*-isomer of formula (IIa) and the *trans-isomer* of formula (IIIa)

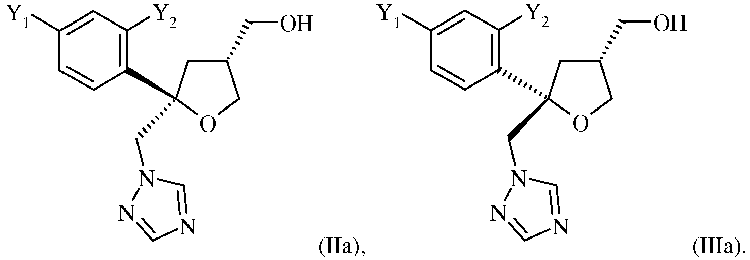

### Providing the compound of formula (I)

No particular restrictions exist as far as providing the compound of formula (I) is concerned.

Generally, it is preferred in step (1) to provide the compound of formula (I) comprised in a solvent system, preferably dissolved in a solvent system. Principally, there are no specific restrictions concerning the solvent system. Suitable solvent systems include, but are not limited to, organic solvent systems comprising at least one ether, at least one cyclic ether, at least one ketone such as methyl-*iso*-butyl-ketone (MIBK), at least one chlorinated solvent such as dichloromethane (DCM), at least one alkane such as pentane or hexane, or mixtures of two or more thereof, in particular solvent systems consisting of at least one ether, at least one cyclic ether, at least one ketone such as methyl-*iso*-butyl-ketone (MIBK), at least one chlorinated solvent such as dichloromethane (DCM), at least one alkane such as pentane or hexane, or mixtures of two or more thereof, and aqueous solvent systems, in particular water. According to one embodiment of the present invention, the compound of formula (I) is provided in said solvent system and is then subjected to the treatment according to step (2). According to another embodiment of the present invention, the compound of formula (I) is provided in step (1) in dry form and is admixed with the solvent system which already contains the halogenation agent employed in step (2).

Preferably, the compound of formula (I) is provided in a solvent system which is a weakly complexing solvent system. Further, it is possible to provide the compound of formula (I) in a solvent system which is not a weakly complexing system, and, in at least one further step, to suitably change the non-weakly complexing solvent system to obtain the compound of formula (I) in a weakly complexing solvent system. The term "weakly complexing solvent" as used in this context of the present invention relates to a solvent system which is only weakly coordinating to acids, (metal) cations or any other compounds present in the solution provided in step (1) of the process according to the present invention. Examples of weakly complexing systems include, but are not limited to, any suitable ethers, in particular ethers like diethylether, tert-butylmethylether, cyclopentylmethylether, tetrahydrofuran, 2-methyltetrahydrofuran, dioxane, and mixtures of two or more of these ethers. According to a preferred embodiment of the present invention, the compound of formula (I) is provided in a solvent system comprising tetrahydrofuran (THF) or comprising THF and at least one further ether compound such as a solvent system comprising THF and at least one further ether selected from the group consisting of diethylether, tert-butylmethylether, cyclopentylmethylether, 2-methyltetrahydrofuran, and dioxane. More preferably, the compound of formula (I) is provided in a solvent system comprising THF as such, i.e. a solvent system consisting of THF.

As mentioned above, it was a particular object of the present invention to provide a new and advantageous process for the separation of the *cis*-isomer of a furan derivative comprising at least one hydroxyl group which can be halogenated, and at least one basic substituent from the respective *trans*-isomer, and a particular object of the present invention to provide a new process for the separation of the *cis*-isomer of formula (IIa), from the *trans*-isomer of formula (IIIa). According to the present invention, it was surprisingly found that such a process can be established if the compound (I), in particular the compound (Ia), is provided in step (1) in above-described weakly complexing solvent system, more preferably in a weakly complexing solvent system comprising THF, more preferably in a weakly complexing solvent system consisting of THF. As discussed hereinbelow, it was found that the use of this solvent system ideally allows for the preparation of the new and advantageous intermediates which in turn allow for the new separation of the *cis*-isomer from the *trans*-isomer.

In step (1) of the process according to the present invention, the compound (I), in particular the compound (Ia), is provided in the solvent system in an amount and in a concentration which is suitable for the further steps of said process. The concentration of the compound (I), in particular of compound (Ia), in said solvent system is preferably in the range of from 0.1 to 1.0 mol/1, more preferably from 0.2 to 0.8 mol/1, more preferably from 0.25 to 0.5 mol/l.

In general, the solvent system according to step (1) of the process according to the present invention, may comprise, in addition to the compound (I), in particular compound (Ia), further components which are suitable and/or necessary for the further steps of said process. In a preferred embodiment, the solvent system of step (1) of the process according to the present invention comprises only the compound (I), in particular the compound (Ia), and further preferably a weakly complexing solvent, preferably tetrahydrofuran (THF), and no further components. Therefore, according to a preferred embodiment of the present invention, step (1) consists in providing the compound (I), in particular the compound (Ia), in a solvent system as described above, wherein said solvent system preferably consists of THF and optionally at least one ether selected from the group consisting of diethylether, tert-butylmethylether, cyclopentylmethylether, 2-methyltetrahydrofuran, and dioxane, said solvent system preferably containing only the compound (I), in particular the compound (Ia).

The solvent system comprising the compound (I), in particular the compound (Ia), is generally provided at any suitable temperature, for example at a temperature in the range of from -30 to +40 °C, preferably from -10 to +20 °C, more preferably from -5 to +5 °C. Preferably, the solvent system comprising the compound (I), in particular the compound (Ia), is provided, in a first step, at a higher temperature, for example in the range of from +20 to +40 °C, preferably from +20 to +30 °C, and is then cooled to a lower temperature, for example to a temperature in the range of from -10 to +10 °C, preferably from -5 to +5 °C.

Preferably, the solvent system comprising the compound (I), in particular the compound (Ia) according to step (1) can be provided under inert gas, for example nitrogen or argon and/or in the absence of water. Preferably, the solvent system comprising the compound (I), in particular the compound (Ia) according to step (1) is conducted under inert gas, for example nitrogen or argon, and in the absence of water.

Preferred compound of formula (Ia) as described above can be provided by any conceivable method. In a particular method, the preferred compound of formula (Ia) can be provided by a method comprising steps (i.1) to (vi.2) as described hereinunder. This specific process is disclosed in order to exemplify a process for the preparation of the compounds according to general formula (I). Compounds according to general formula (I) can also be prepared according to this process comprising steps (i.1) to (vi.2), wherein it is clearly understandable for a person having ordinary skill in the art that the residues R¹, R², R³, R⁴, R⁵ and B will be chosen accordingly.

The compound of formula (Ia) can also be provided as an at least partially crystalline compound by a method comprising, in addition to steps (i.1) to (vi.2), a further step (vii) of at least partially crystallizing the compound of formula (Ia). The at least partially crystalline compound thus obtained is then preferably admixed with the solvent system as described above, in particular with the solvent system comprising THF, more preferably with the solvent system consisting of THF.

### Steps (i.1) to (vii)

As mentioned, the preferred compound of formula (Ia) can be provided by a method comprising
(i.1) reacting a compound of formula (A) 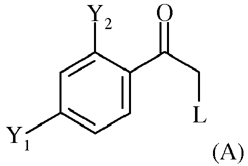 wherein L is a leaving group, preferably a halogen, more preferably Cl, in a solvent with a nucleophilic compound comprising a nucleophilic residue RₐR_{b}R_{c}Si-CH₂ wherein Rₐ, R_{b} and R_{c} are the same or different and selected from the group consisting of optionally suitably substituted alkyl and aryl residues, to obtain a reaction mixture containing as intermediate a beta-hydroxy silane of formula 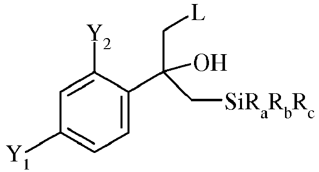 said reacting preferably being performed at a temperature in the range of from -50 to +20°C, more preferably from -30 to +10°C, more preferably from -15 to +5°C; (i.2) treating the resulting reaction mixture, preferably without change of solvent, with a reagent promoting elimination reaction to obtain a reaction mixture containing a compound of formula (B) 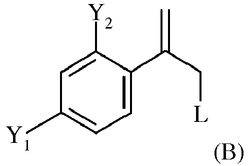 wherein treating is performed at a temperature in the range of from -20 to +70 °C and wherein said reagent is preferably an acid, preferably an inorganic acid, more preferably sulfuric acid, wherein, if sulfuric acid is used, the temperature at which said treating is performed is preferably in the range of from +40 to +50°C;
(ii) reacting the compound of formula (B) with a malonic ester R_{d}OOC-CH₂-COORₑ to obtain a compound of formula (C) 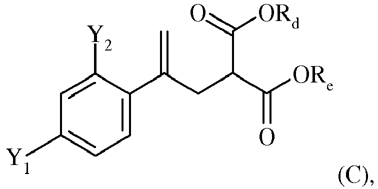 wherein R_{d} and Rₑ are independently an optionally suitably substituted alkyl group having from 1 to 5 carbon atoms, preferably ethyl, wherein, after (ii) and before (iii), the compound of formula (C) is optionally separated by extraction in a suitable solvent, preferably cyclohexane;
(iii) reducing the compound of formula (C) to obtain a compound of formula (D) 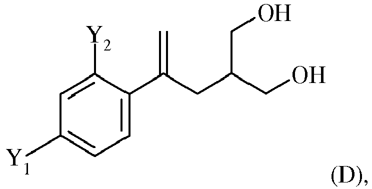 the reducing agent preferably being LiBH₄ which is used in an amount of at most 2 molar equivalents with respect to the compound of formula (C), said reduction preferably being carried out in a suitable solvent preferably comprising water, the solvent preferably being selected from the group consisting of water, alcohol, and a mixture of water and at least one alcohol, more preferably from the group consisting of water, methanol, ethanol, isopropanol, and a mixture of water and at least one of these alcohols, more preferably from the group consisting of water, ethanol, isopropanol, and a mixture of water and at least one of these alcohols, more preferably from the group consisting of water, isopropanol, and a mixture of water and isopropanol, the solvent most preferably being a mixture of water and isopropanol, wherein the solvent preferably comprises from 1 to 20 vol.-%, more preferably from 5 to 15 vol.-% of water;
(iv) acylating the compound of formula (D) with isobutyric anhydride to obtain a compound of formula (E) 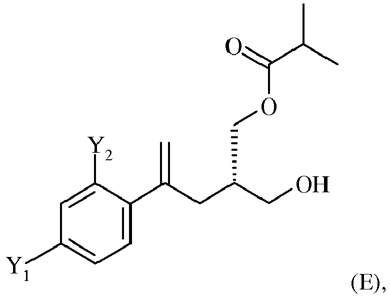 said acylation preferably being carried out in the presence of a suitable enzyme, preferably Novo SP 435 enzyme in a suitable solvent, preferably acetonitrile or toluene, more preferably toluene, wherein after (iv) and before (v), the compound of formula (E) is preferably at least partially crystallized;
(v) reacting the compound of formula (E) with a halogen Hal₂ selected from the group consisting of Cl₂, Br₂ and I₂, preferably I₂, in the presence of a base in a solvent to obtain a compound of formula (F) 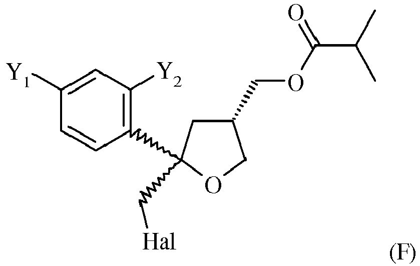 wherein preferably from 80 to 95 %, more preferably from 85 to 95 % of the molecules of compound (F) are present as *cis*-isomer of formula (Fa) 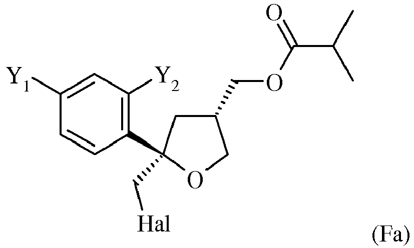 and preferably from 20 to 5 %, more preferably from 15 to 5 % of the molecules of compound (F) are present as *trans*-isomer of formula (Fb) 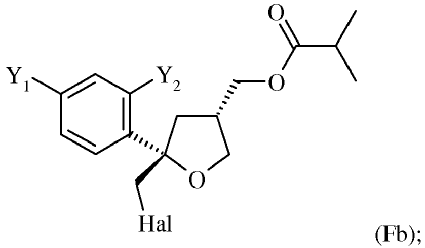 wherein the solvent is preferably ethyl acetate and wherein the base is preferably sodium hydrogencarbonate, and wherein the temperature at which the compound of formula (E) is reacted is preferably less than 0 °C, more preferably not higher than -5 °C and even more preferably not higher than -10 °C;
(vi. 1) heating the compound of formula (F) preferably at a temperature in the range of from +70 to +100 °C, more preferably from +80 to +95 °C, more preferably from +85 to +90 °C, preferably in the absence of DMPU (1,3-dimethyl-3,4,5,6-tetrahydro-2(1H)-pyrimidinone), in a solvent, preferably a polar aprotic solvent, for example DMF (*N,N*-dimethylformamide) and DMSO (dimethyl sulfoxide), more preferably DMSO with a 1,2,4-triazole alkali metal salt, preferably the sodium salt, and treating the resulting reaction mixture with a base suitable to promote saponification of the ester moiety such as alkali metal hydroxides, alkali metal bicarbonates, alkali metal carbonates, alkaline earth metal hydroxides, alkaline earth metal bicarbonates, and alkaline earth metal carbonates, preferably alkali metal bases, said base preferably being added in aqueous and/or alcoholic media, wherein suitable alcohols are alcohols containing 1 to 6, preferably 1 to 4, more preferably 1 to 3, most preferably 1 to 2 carbon atoms, said base even more preferably being sodium hydroxide, preferably employed as aqueous solution, in the presence of methanol, to obtain the compound of formula (Ia) 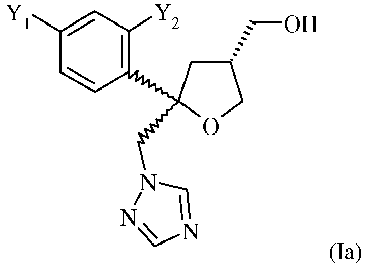 wherein preferably from 80 to 95 %, more preferably from 85 to 95 % of the molecules are present as *cis*-isomer of formula (IIa) 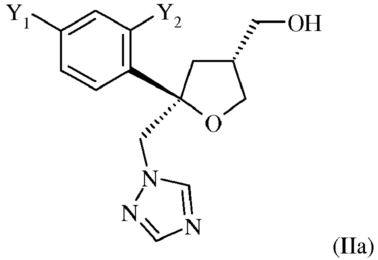 and preferably from 20 to 5 %, more preferably from 15 to 5 % of the molecules are present as *trans*-isomer of formula (IIIa) 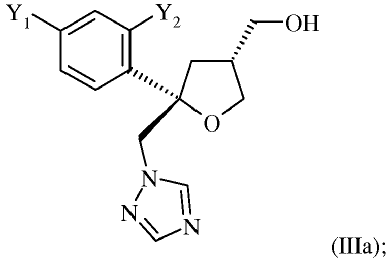
(vi.2) separating the compound of formula (Ia) from the reaction mixture obtained from (vi.1) by extraction in a suitable solvent, the solvent preferably being a polar water-immiscible solvent, more preferably an ester such as ethyl acetate or isopropyl acetate, an ether such as tetrahydrofuran or methyl tetrahydrofuran, a ketone such as methyl isobutyl ketone, a halogenated solvent such as dichloromethane, toluene, or a mixture of two or more of these solvents, more preferably an ester or an ether, more preferably an ether, and even more preferably methyl tetrahydrofuran.

### Steps (i.1) to (vi.2) in detail

In step (i.1) of the herein-mentioned process, the compound of formula (A) comprises residues Y₁ and Y₂, wherein Y₁ and Y₂ are independently F or Cl. Thus, Y₁ may be F or Cl, and independently from the chemical nature of Y₁, Y₂ may be F or Cl. Preferably, both Y₁ and Y₂ are either F or Cl. More preferably, both Y₁ and Y₂ are F.

The term "leaving group L" as used in the context of step (i.1) refers to any chemical moiety L which, under suitable reaction conditions, departs from compound (A) with a pair of electrons in a heterolytic bond cleavage. For this purpose, compound (A) as used herein may comprise any suitable leaving group L. Preferably, the leaving group L, after departing, is a neutral or an anionic moiety, more preferably an anionic moiety. Even more preferably, L is a halogen such as, for example, Cl, Br, I. Preferably, L is Cl.

The nucleophilic compound with which compound (A) is reacted in step (i.1) comprises a nucleophilic residue RₐR_{b}R_{c}Si-CH₂. As to the chemical nature of this residue, there are no particular restrictions provided that the beta-hydroxy silane intermediate of formula 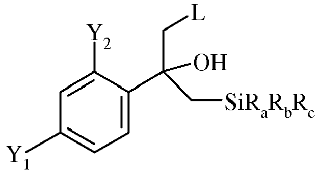 is obtained. The term "intermediate" as used in this context generally refers to a beta-hydroxy silane which is comprised in the reaction mixture obtained in step (i.1) and which is formed from the reactants of step (i.1) and reacts further in (i.2). The term "intermediate" as used in this context does not exclude such beta-hydroxy silanes which can be isolated from the reaction mixture obtained in (i.1).

The nucleophilic compound employed in (i.1) can be any suitable compound comprising a nucleophilic residue RₐR_{b}R_{c}Si-CH₂ which, when reacted with compound (A), either directly or indirectly leads to the formation of the beta-hydroxy silane intermediate discussed above. Rₐ, R_{b} and R_{c} comprised in the nucleophilic compound are the same or different and selected from the group consisting of optionally suitably substituted alkyl and aryl residues. The term "optionally suitably substituted aryl residue" as used in this context refers to aryl residues which have, for example, up to 6 or up to 12 carbon atoms. If such aryl residue is a substituted aryl residue, the number of carbon atoms refers to the number of carbon atoms of the corresponding unsubstituted aryl residue. The term "optionally suitably substituted alkyl residue" as used in this context refers to alkyl residues which have, for example, 1 to 20, preferably 1 to 10 carbon atoms. If such alkyl residue is a substituted alkyl residue, the number of carbon atoms refers to the number of carbon atoms of the corresponding unsubstituted alkyl residue.

Preferably, Rₐ, R_{b} and R_{c} comprised in the nucleophilic compound are the same or different and selected from the group consisting of alkyl residues, more preferably non-substituted alkyl residues having from 1 to 6 carbon atoms, preferably from 1 to 4 carbon atoms such as methyl, ethyl, n-propyl, iso-propyl, n-butyl, iso-butyl, and tert-butyl, more preferably 1 or 2 carbon atoms, methyl or ethyl, with Rₐ, R_{b} and R_{c} in particular being methyl.

Preferably, the nucleophilic compound employed in (i.1) is a Grignard reagent. The term "Grignard reagent" as used in this context refers to any suitable nucleophilic organometallic reagent comprising the nucleophilic residue RₐR_{b}R_{c}Si-CH₂. Preferably the nucleophilic compound is a Grignard compound RₐR_{b}R_{c}Si-CH₂MgX wherein X is a suitable anionic species which is preferably selected from the group consisting of Cl, Br, and I. More preferably, the Grignard compound is the compound RₐR_{b}R_{c}Si-CH₂MgCl.

As solvent which is employed in (i.1), any solvent or solvent mixture is conceivable, preferably a solvent or solvent mixture in which a Grignard reaction can be carried out. Suitable solvents are, for example, ether compounds such as diethyl ether and/or tetrahydrofuran (THF), or MTBE, preferably MTBE (methyl-tert-butyl-ether).

Preferably, in the process as defined above, in (i.1), the compound of formula (A) is the compound (Aa) 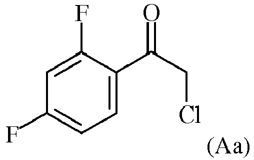 which is reacted in MTBE as solvent with the nucleophilic compound (H₃C)₃Si-CH₂MgCl to obtain a reaction mixture containing as intermediate a beta-hydroxy silane of the formula:

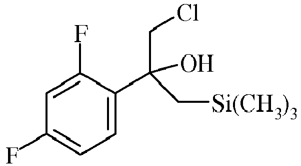

As to the temperatures at which the reaction in (i.1) is carried out, no particular restrictions exist provided a reaction mixture is obtained which allows for the reaction in (i.2). Preferably, reacting in (i.1) is performed at a temperature in the range of from -50 to +20 °C, more preferably from -40 to +15 °C, more preferably from -30 to +10 °C, more preferably from -20 to +10 °C, more preferably from -15 to +5 °C such as at a temperature in the range of from -15 to -10 °C or from -10 to -5 °C or from -5 to 0 °C or from 0 to +5 °C.

The reaction mixture resulting from (i.1) is treated in (i.2), preferably without change of solvent, with a reagent promoting elimination reaction to obtain a reaction mixture containing a compound of formula (B)

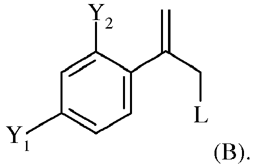

Carrying out the present process without solvent exchange after (i.1) is particularly preferred if MTBE is used as solvent in (i.1).

As to the temperatures at which the reaction in (i.2) is carried out, no particular restrictions exist provided a reaction mixture is obtained containing the compound of formula (B). Preferably, treating in (i.2) is performed at a temperature in the range of from -20 to +70 °C. Preferred temperature ranges are, for example, -20 to -10 °C or -10 to 0 °C or 0 to +10 °C or +10 to +20 °C or +20 to +30 °C or +30 to +40 °C or +40 to +50 °C or +50 to +60 °C or +60 to +70 °C.

As to the reagent promoting elimination reaction employed in (i.2), no particular restrictions exist provided that the compound of formula (B) is obtained, preferably without solvent exchange after (i.1). Preferably, the reagent is an acid or a mixture of two or more acids. More preferably, the reagent is an inorganic acid or a mixture of two or more inorganic acids. Especially preferred is the use of sulfuric acid. Preferably, if sulfuric acid is used as reagent, the temperature at which (i.2) is performed is in the range of from +40 to +50 °C.

Therefore, in the process as defined above, in (i.2), the reaction mixture resulting from (i.1) is treated without change of solvent with sulfuric acid promoting elimination reaction to obtain a reaction mixture containing, as compound of formula (B), the compound (Ba):

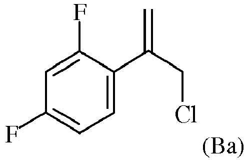

Thus, the process as defined above comprises
(i.1) reacting a compound of formula (Aa) 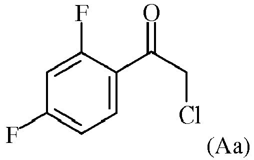 with (H₃C)₃Si-CH₂MgCl in MTBE as solvent to obtain a reaction mixture containing as intermediate a beta-hydroxy silane of formula 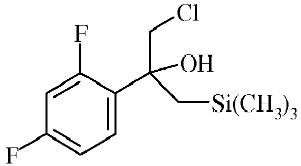
(i.2) treating the resulting reaction mixture without change of the solvent MTBE with sulfuric acid promoting elimination reaction to obtain a reaction mixture containing a compound of formula (Ba) 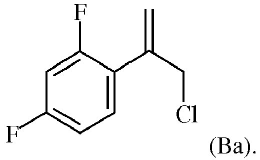 In particular, the process as defined above comprises
(i.1) reacting a compound of formula (Aa) 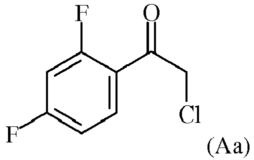 with (H₃C)₃Si-CH₂MgCl in MTBE as solvent at a temperature in the range of from -15 to +5 °C to obtain a reaction mixture containing as intermediate a beta-hydroxy silane of formula 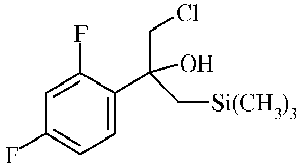
(i.2) treating the resulting reaction mixture without change of the solvent MTBE at a temperature in the range of from +40 to +50 °C with sulfuric acid promoting elimination reaction to obtain a reaction mixture containing a compound of formula (Ba)

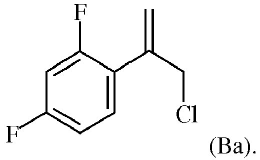

According to step (ii), the compound of formula (B) is preferably reacted with a malonic ester R_{d}OOC-CH₂-COORₑ wherein R_{d} and Rₑ are independently an optionally suitably substituted alkyl group having from 1 to 5 carbon atoms. The number of carbon atoms refers to the number of carbon atoms of the unsubstituted alkyl residue. Preferred alkyl groups R_{d} and Rₑ have 1 to 4 carbon atoms, such as methyl, ethyl, n-propyl, iso-propyl, n-butyl, iso-butyl, and tert-butyl. Even more preferably the alkyl groups R_{d} and Rₑ have 1 or 2 carbon atoms, such as methyl or ethyl, with ethyl being especially preferred. Even more preferably, the alkyl groups R_{d} and Rₑ are unsubstituted alkyl groups.

In step (ii), it is further preferred to react the malonic ester R_{d}OOC-CH₂-COORₑ with compound (B) in the presence of a suitable strong base, preferably a strong alkali metal base allowing for the reaction of the respective anion ⁻CH(COOR_{d})(COORₑ) derived from the malonic ester R_{d}OOC-CH₂-COORₑ. As alkali metal, sodium is preferred. Suitable bases are, for example, NaH or NaOH, with NaOH being preferred. NaOH can be employed in every suitable form. Preferably, NaOH is employed as solid, such as, for example, in the form of NaOH flakes. The solvent in which step (ii) is carried out can be chosen according to, for example, the specific chemical nature of the strong base as discussed above. Suitable solvents are, for example, THF, DMSO or the like, wherein DMSO is preferred. The temperatures at which the reaction in step (ii) is carried out can be chosen in accordance with the solvent and the base. Preferred temperatures are in the range of from 0 to +35 °C, more preferably from +25 to +30 °C.

The product of the reaction in (ii), the compound of formula (C) 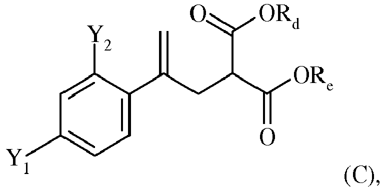 is preferably suitably separated from the reaction mixture obtained in (ii). Preferably, this separation includes a step wherein the compound (C) is separated by extraction in a suitable solvent, wherein cyclohexane is preferred.

The organic layer obtained from extraction can be washed in one or more steps. As washing agents, water and aqueous basic solutions such as, for example, aqueous solutions of alkali metal bases such as alkali metal hydroxide, preferably sodium hydroxide, are to be mentioned.

Preferably, the compound of formula (C) obtained from step (ii) is suitably reduced in step (iii) wherefrom a compound of formula (D) is obtained:

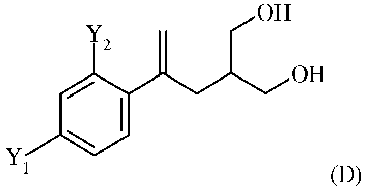

Reducing in step (iii) can be carried out according to any suitable method involving any suitable reducing agent, wherein the use of a hydride reducing agent is preferred. Such hydride reducing agent is, for example, sodium borohydride (NaBH₄), lithium borohydride (LiBH₄), lithium aluminium hydride (LiAlH₄), diisobutylaluminium hydride (DIBAL) or lithium triethylborohydride (LiEt₃BH), wherein LiBH₄ is preferably employed as reducing agent in step (iii), preferably in an amount of at most 2 molar equivalents of LiBH₄ with regard to the compound of formula (C).

As to the solvent in which the reaction of step (iii) is carried out, no particular restrictions exist provided that the compound of formula (D) is obtained. Preferred solvents are selected from the group consisting of water, alcohol, and a mixture of water and at least one alcohol. Preferred alcohols are methanol, ethanol, and isopropanol. Therefore, the solvent is preferably selected from the group consisting of water, methanol, ethanol, isopropanol, and a mixture of water and at least one of these alcohols, more preferably from the group consisting of water, ethanol, isopropanol, and a mixture of water and at least one of these alcohols, more preferably from the group consisting of water, isopropanol, and a mixture of water and isopropanol.

For the most preferred reducing agent used in step (iii), LiBH₄, a mixture of water and isopropanol is the most advantageous solvent.

Therefore, the solvent used in step (iii) preferably comprises water, wherein the solvent more preferably comprises from 1 to 20 vol.-%, more preferably from 5 to 15 vol.-% of water.

The temperatures at which the reaction in step (iii) is carried out can be chosen in accordance with the solvent and the reducing agent. Preferred temperatures are in the range of from 0 to +40 °C, more preferably from +20 to +35 °C, more preferably from +25 to +30 °C.

The product of the reduction in (iii), the compound of formula (D), is preferably suitably separated from the reaction mixture obtained in (iii). Preferably, this separation includes a step wherein the compound (D) is separated by extraction in a suitable solvent. Among the suitable solvents, toluene is preferred.

According to step (iv), the compound of formula (D) is preferably acylated with isobutyric anhydride to obtain a compound of formula (E)

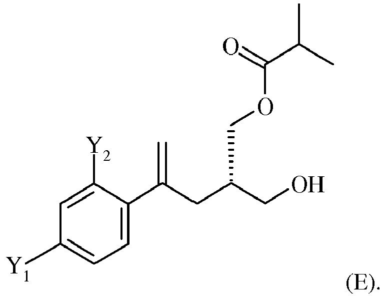

More preferably, acylation in (iv) is carried out in the presence of a suitable enzyme, preferably Novo SP 435 enzyme in a suitable solvent, preferably acetonitrile or toluene, more preferably toluene, e.g. analogously to the method described in WO 97/22710. The choice of toluene as solvent is also beneficial in extractive work up as no additional solvent is required. In case of acetonitrile as solvent it is required to use an additional immiscible solvent for extractive work up.

The temperatures at which the acylation in step (iv) is carried out can be chosen in accordance with the solvent, the acylation agent and the enzyme. Preferred temperatures are in the range of from -20 to -5 °C, more preferably from -15 to -10 °C.

The obtained reaction mixture is preferably further treated with a suitable base such as, for example, sodium hydrogencarbonate.

Preferably, the compound of formula (E) is suitably at least partially crystallized from the reaction mixture. Crystallization can be carried out according to any conceivable method. Preferably, the compound of formula (E) is crystallized from n-heptane.

According to step (v), the compound of formula (E) is preferably reacted with a halogen Hal₂ selected from the group consisting of Cl₂, Br₂ and I₂, preferably I₂, in the presence of a base in a solvent to obtain a compound of formula (Fa)

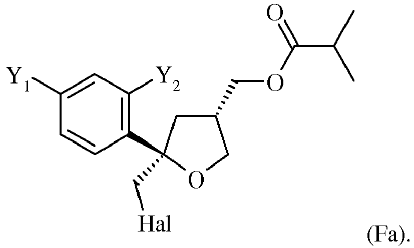

Generally, it is possible to carry out the reaction in step (v) in the presence of a base such as pyridine and in a suitable solvent such as acetonitrile, THF, EtOAc (ethyl acetate) or CH₂Cl₂ (DCM) at a temperature in the range of from -20 to +30 °C. Reference is made to WO 94/25452 A1, pages 16 and 35. The reaction is suitably carried out in ethyl acetate as solvent wherein as base, sodium hydrogencarbonate is employed. Further, the temperature for carrying out the reaction is preferably less than 0 °C, more preferably not higher than -5 °C and even more preferably not higher than -10 °C.

After the reaction, the organic layer, optionally after suitable quenching, may be optionally washed at least once.

Preferably, the *cis*-isomer of formula (Fa) is obtained in step (v) together with the compound of formula (Fb), the respective *trans-*isomer

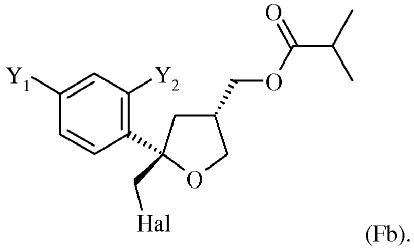

This mixture of the compounds of formula (Fa) and (Fb) is referred to in the following as compound of formula (F)

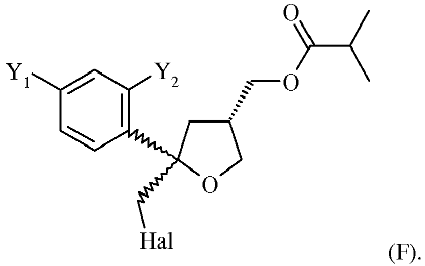

In said compound (F), preferably from 80 to 95 %, more preferably from 85 to 95 % of the molecules are present as *cis*-isomer of formula (Fa) and preferably from 20 to 5 %, more preferably from 15 to 5 % of the molecules of compound (F) are present as *trans*-isomer of formula (Fb).

According to step (vi.1) of the present process, the compound of formula (F), i.e. in particular the compound of formula (Fa) and the compound of formula (Fb), is preferably suitably heated in a suitable solvent with a suitable 1,2,4-triazole salt. Preferred 1,2,4-triazole salts are alkali metal salts, with the sodium salt being especially preferred. Preferred solvents are polar aprotic solvents, for example, DMF (*N,N*-dimethylformamide) and DMSO, with DMSO being preferred.

The temperature to which the reaction mixture in step (vi.1) is heated is preferably in the range of from +70 to +100 °C, preferably from +80 to +95 °C and more preferably from +85 to +90 °C.

The mixture obtained from heating is then preferably treated with a suitable base to promote saponification of the ester moiety. Such bases are, for example, alkali metal hydroxides, alkali metal bicarbonates, alkali metal carbonates, alkaline earth metal hydroxides, alkaline earth metal bicarbonates, and alkaline earth metal carbonates. The alkali metal bases are preferred. Preferably, the base is added in aqueous and/or alcoholic media. Suitable alcohols are alcohols containing 1 to 6, preferably 1 to 4, more preferably 1 to 3, most preferably 1 to 2 carbon atoms. The preferred base is sodium hydroxide, preferably employed as aqueous solution, in the presence of methanol.

In step (vi.1), preferably the compound of formula (Ia) 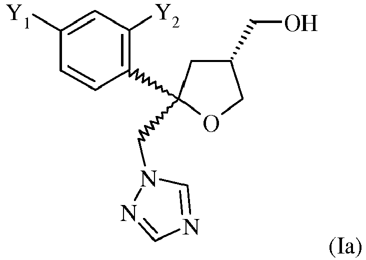 is obtained, wherein preferably from 80 to 95 %, more preferably from 85 to 95 % of the molecules are present as *cis*-isomer of formula (IIa) 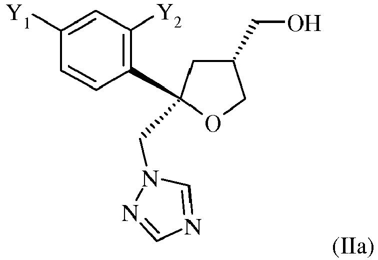 and preferably from 20 to 5 %, more preferably from 15 to 5 % of the molecules are present as *trans*-isomer of formula (IIIa)

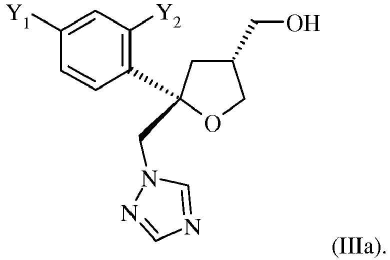

According to the present process, the compound of formula (Ia) is separated from the reaction mixture obtained from (vi.1) by extraction in a suitable solvent according to step (vi.2). Reference is made to the respective discussion hereinabove.

According to optional step (vii) of the present process, the compound of formula (Ia) is at least partially crystallized. Preferably, the compound of formula (Ia) is crystallized from a solvent, optionally by addition of an antisolvent, wherein the solvent is preferably the solvent or the solvent mixture employed in (vi.2) and wherein the antisolvent is preferably a saturated or unsaturated hydrocarbon such as cyclohexane, hexane, or heptanes, or a mixture of two or more thereof.

After crystallization, the crystallized compound (Ia) is preferably separated from its mother liquor, for example by suitable filtration, and preferably washed at least once with a suitable washing agent. Preferred washing agents are the solvent mixture used for the crystallization and the antisolvent discussed above. After such preferred separation, the crystallized compound (Ia) can be dried under suitable drying conditions. Drying in vacuo is preferred, wherein the temperatures are preferably in the range of from +20 to +50 °C, more preferably from +30 to +45 °C.

### Step (2)

Step (2) of the process according to the present invention comprises treating the compound of formula (I), preferably comprised in the weakly complexing solvent system, with at least one halogenation system to obtain a compound of formula (XI) 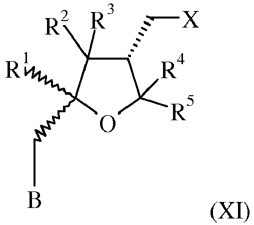 or its corresponding HX salt, containing the *cis*-isomer of formula (XII) 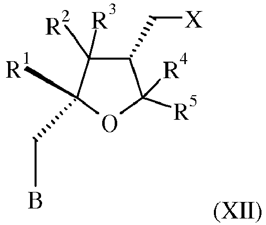 or its corresponding HX salt and the *trans-isomer* of formula (XIII) 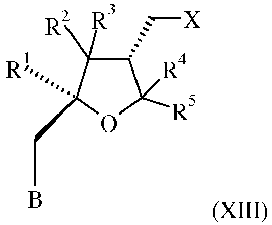 or its corresponding HX salt; wherein X is Cl, Br, or I.

Generally, it is conceivable to treat the compound of formula (I), preferably (Ia), with at least one chlorination agent, such as thionyl chloride, at least one bromination agent, such as thionyl bromide or HBr, or with at least one iodination agent, such as I₂ together with triphenylphosphine (PPh₃).

In these cases, it is possible to provide the compound of formula (I), preferably (Ia), in step (1) in a solvent system which is identical to the solvent system in which the halogenation agent, i.e. the chlorination agent, the bromination agent or the iodination agent is employed. For example, the halogenation agent can be employed as aqueous solution. Therefore, as to these cases, it is possible to provide the compound of formula (I), preferably (Ia), in step (1) in an aqueous solvent system, preferably water.

### X=Br, I

According to a first embodiment, the compound of formula (I), preferably (Ia), is treated in step (2) with at least one bromination agent.

According to a first alternative of this first embodiment, the solvent system which is used in step (1) and the solvent system in which the bromination agent is employed, are preferably the same. Preferably, an aqueous solution of HBr is used. More preferably, the compound of formula (I), preferably (Ia), in its dried form is admixed with the aqueous solution of HBr, preferably at a temperature in the range of from +10 to +40 °C, more preferably from +20 to +30 °C. Admixing can be carried out under an inert atmosphere, for example under an atmosphere of nitrogen or argon, or in air or lean air, with air being preferred. The concentration of the reaction mixture with regard to the compound of formula (I), preferably (Ia), may vary between 0.1 and 4 mol/1, and is preferably in the range of from 0.7 to 1.5 mol/l. It is further preferred to add, to the resulting reaction mixture, a strong hygroscopic acid such as H₂SO₄. The acid is suitably added in an amount so that the molar ratio of the compound of formula (I), preferably (Ia), and the acid is in the range of from 0.3 to 0.8, preferably from 0.4 to 0.6. Treating the compound of formula (I), preferably (Ia), in step (2) with the bromination agent is preferably carried out at elevated temperatures, preferably at a temperature of +10 to +150 °C, more preferably from +80 to +125 °C. In particular, treating is carried out under reflux conditions. In the course of the treatment, it is preferred to suitably stir the reaction mixture. After the desired conversion, preferably after completion of the bromination reaction, heating is stopped and the resulting reaction mixture is cooled in one or more steps to a temperature of at most +30 °C, preferably at most +20 °C, more preferably in the range of from -20 to +20 °C, more preferably from -10 to +10 °C, more preferably from -5 to +5 °C. The work-up of the obtained cooled reaction mixture can be conducted according to methods known by the skilled person. Preferably, the resulting aqueous layer is suitably extracted with at least one solvent such as dichloromethane (DCM). The combined organic layers are preferably dried with at least one suitable drying agent such as sodium sulfate or the like, followed by removal of the solvent, for example by evaporation, preferably under reduced pressure. The thus obtained product is preferably subjected to at least one suitable purification stage, for example by chromatographic methods such as flash chromatography. According to this embodiment, the compound of formula (XI), preferably (XIa) is obtained wherein X = Br, said compound of formula (XI), preferably (XIa) containing the *cis*-isomer of formula (XII), preferably (XIIa), and the *trans*-isomer of formula (XIII), preferably (XIIIa), in essentially the same *cis: trans* ratio as the compound of formula (I), preferably (Ia), contains the *cis*-isomer of formula (II), preferably (IIa), and the *trans*-isomer of formula (III), preferably (IIIa).

According to a second alternative of this first embodiment, the solvent system which is used in step (1) is a weakly complexing solvent system as defined above, preferably a weakly complexing solvent system comprising, more preferably consisting of, an ether, more preferably MTBE (methyl-*tert*-butyl-ether). Further preferably, the compound of formula (I), preferably (Ia), is provided in its dry form and admixed with a mixture comprising the bromination agent, preferably thionyl bromide, and the solvent system, preferably at a temperature in the range of from -10 to +15 °C, more preferably from -5 to +10 °C, more preferably from 0 to +5 °C. Admixing can be carried out under an inert atmosphere, for example under an atmosphere of nitrogen or argon, or in air or lean air, with air being preferred. The concentration of the reaction mixture with regard to the compound of formula (I), preferably (Ia), may vary between 0.1 and 4 mol/l, and is preferably in the range of from 0.7 to 1.5 mol/l. Treating the compound of formula (I), preferably (Ia), in step (2) with the bromination agent according to the second alternative is preferably carried out at elevated temperatures, preferably under reflux conditions. In the course of the treatment, it is preferred to suitably stir the reaction mixture. After the desired conversion, preferably after completion of the bromination reaction, heating is stopped and the resulting reaction mixture is cooled in one or more steps to a temperature of at most +30 °C, preferably at most +20 °C, more preferably in the range of from -20 to +20 °C, more preferably from -10 to +10 °C, more preferably from -5 to +5 °C. The work-up of the obtained cooled reaction mixture can be conducted according to methods known by the skilled person. Preferably, the solid obtained from step (2) is suitably separated, for example by filtration, centrifugation or decantation, preferably filtration, and the thus obtained solid is then preferably washed at least once with at least one suitable washing agent, preferably a solvent comprised in the solvent system used in step (1) and (2), more preferably the solvent system used in step (1) and (2). Thereafter, the preferably washed solid can be suitably dried, for example at reduced pressure at elevated or ambient temperature, preferably ambient temperature. Optionally, the thus obtained product is subjected to at least one suitable purification, for example by chromatographic methods such as flash chromatography. According to this embodiment, the HBr salt of the compound of formula (XI), preferably (XIa) is obtained wherein X = Br, said HBr salt of the compound of formula (XI), preferably (XIa) containing the HBr salt of the *cis*-isomer of formula (XII), preferably (XIIa), and the HBr salt of the *trans*-isomer of formula (XIII), preferably (XIIIa), in essentially the same *cis:trans* ratio as the compound of formula (I), preferably (Ia), contained the *cis*-isomer of formula (II), preferably (IIa), and the *trans-*isomer of formula (III), preferably (IIIa).

According to a second embodiment, the compound of formula (I), preferably (Ia), is treated in step (2) with at least one iodination agent. Generally, it is conceivable to carry out this treatment essentially in accordance with the procedure as described hereinabove regarding the treatment with at least one bromination agent according to the first embodiment. Preferably, in case the compound (I), preferably (Ia), is to be iodized, the compound (I), preferably (Ia), is subjected to chlorination in a first stage and, in a second stage, subjected to a substitution reaction wherein Cl is substituted by I. Concerning the chlorination reaction, reference can be made, for example, to the chlorination reaction described hereinunder in the first alternative of the third embodiment. Starting from the compound (IV), 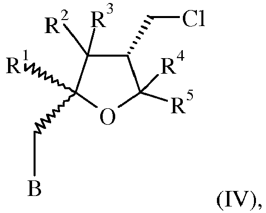 preferably (IVa) 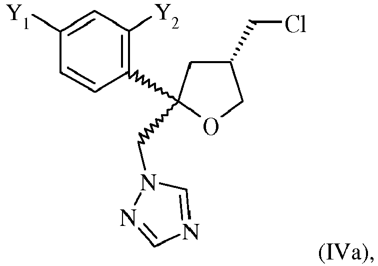 it is preferred to dissolve this compound in a preferably organic solvent, for example methyl-*iso*-butyl-ketone (MIBK). The iodination agent that is used to substitute the Cl atom can be selected from the known iodination agents, for example alkali metal iodides and earth alkaline metal iodides, preferably sodium iodide (NaI) and potassium iodide (KI) or mixtures thereof. Admixing the compound (IV), preferably (IVa), in the solvent with the iodination agent can be carried out under an inert atmosphere, for example under an atmosphere of nitrogen or argon, or in air or lean air, with air being preferred, the temperature being in the range of from +10 to +40 °C, more preferably from +20 to +30 °C. The concentration of the reaction mixture with regard to the compound of formula (IV), preferably (IVa), may vary between 0.005 and 0.05 mol/1, and is preferably in the range of from 0.01 to 0.05 mol/l. Treating the compound of formula (IV), preferably (IVa), in step (2) with the iodination agent is preferably carried out at elevated temperatures, preferably under reflux conditions. In the course of the treatment, it is preferred to suitably stir the reaction mixture. After the desired conversion, preferably after completion of the iodination reaction, heating is stopped and the resulting reaction mixture is cooled in one or more steps to a temperature of at most +30 °C, preferably at most +20 °C. The work-up of the obtained cooled reaction mixture can be conducted according to methods known by the skilled person. Preferably, the resulting reaction mixture is suitably concentrated, for example by evaporation under reduced pressure, and partitioning the resulting concentrate in an aqueous layer and an organic phase, wherein any suitable organic solvent such as DCM can be employed. The organic layer is preferably dried with at least one suitable drying agent such as sodium sulfate or the like, followed by removal of the solvent, for example by evaporation, preferably under reduced pressure. The thus obtained product can be, if desired, subjected to at least one suitable purification stage, for example by chromatographic methods such as flash chromatography. According to this embodiment, the compound of formula (XI), preferably (XIa) is obtained wherein X = I, said compound of formula (XI), preferably (XIa) containing the *cis*-isomer of formula (XII), preferably (XIIa), and the *trans*-isomer of formula (XIII), preferably (XIIIa), in essentially the same *cis: trans* ratio as the compound of formula (I), preferably (Ia), contains the *cis*-isomer of formula (II), preferably (IIa), and the *trans*-isomer of formula (III), preferably (IIIa).

X=Cl

According to a third, and preferred, embodiment of the present invention, the compound of formula (I), preferably (Ia), is treated with at least one suitable chlorination agent, more preferably with at least one chlorination agent which is derived from an acid and which is capable of releasing HCl *in situ* during chlorination.

According to a first alternative of this third embodiment, the solvent system which is used in step (1) is a non-weakly complexing solvent system as defined above, preferably a non-weakly complexing solvent system comprising, preferably consisting of, at least one organic solvent, more preferably at least one alkane and at least one chlorinated alkane, more preferably hexane and DCM (dichloromethane). More preferably, the compound of formula (I), preferably (Ia), in its dried form is admixed with the solvent system, preferably at a temperature in the range of from -10 to +15 °C, more preferably from -5 to +10 °C, more preferably from 0 to +5 °C. Admixing can be carried out under an inert atmosphere, for example under an atmosphere of nitrogen or argon, or in air or lean air, with air being preferred. The concentration of the reaction mixture with regard to the compound of formula (I), preferably (Ia), may vary between 0.1 and 4 mol/l. Treating the compound of formula (I), preferably (Ia), in step (2) with the chlorination agent, preferably thionyl chloride, is preferably carried out at elevated temperatures, preferably at a temperature of from +30 to +100 °C, more preferably from +30 to +80 °C. In particular, treating is carried out under reflux conditions. In the course of the treatment, it is preferred to suitably stir the reaction mixture. After the desired conversion, preferably after completion of the chlorination reaction, heating is stopped and the resulting reaction mixture is cooled in one or more steps to a temperature of at most +40 °C, preferably at most +30 °C, more preferably in the range of from +20 to +30 °C. The work-up of the obtained cooled reaction mixture can be conducted according to methods known by the skilled person. Preferably, the resulting reaction mixture is subjected to a suitable extraction, separation, and suitable washing of the organic layer. After a preferred drying of the thus obtained washed organic layer with at least one suitable drying agent such as sodium sulfate or the like, solvent is suitably removed, for example by evaporation under reduced pressure of at most 100 mbar, preferably at most 50 mbar and at elevated temperatures in a preferred range of from +35 to +55 °C, preferably from +40 to +50 °C. According to this preferred embodiment, the compound of formula (XI), preferably (XIa) is obtained wherein X = Cl, said compound of formula (XI), preferably (XIa) containing the *cis*-isomer of formula (XII), preferably (XIIa), and the *trans*-isomer of formula (XIII), preferably (XIIIa), in essentially the same *cis:trans* ratio as the compound of formula (I), preferably (Ia), contains the *cis*-isomer of formula (II), preferably (IIa), and the *trans*-isomer of formula (III), preferably (IIIa).

Preferred step (2), preparation of the HX salt of the compound of formula (XI) with X = Cl

According to a second alternative of this third embodiment which is an especially preferred embodiment of the present invention, the compound of formula (I), preferably (Ia), is treated with at least one suitable chlorination agent wherein the solvent system comprising the compound (I), preferably (Ia), according to step (1) is the weakly complexing solvent system as defined above, in particular the solvent system comprising THF, more preferably consisting of THF.

As to the chlorination agent used in step (2), derived from an acid and being capable of releasing HCl *in situ* during chlorination, generally any chlorination agent may be used if it fulfills the demands as mentioned above. Examples of suitable chlorination agents which can be used in step (2) of the process according to the present invention are selected from the group consisting of SOCl₂, PCl₃, PCl₅, POCl₃, (COCl)₂ and mixtures of two or more thereof. In a preferred embodiment of the process according to the present invention, the chlorination agent derived from an acid and being capable of releasing HCl *in situ* during chlorination is thionyl chloride (SOCl₂). Thionyl chloride (SOCl₂) according to the following formula 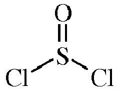 is known to the skilled person. Thionyl chloride can be prepared by known methods and is commercially available.

Therefore, the present invention also relates to the process as defined above, wherein in step (2), the chlorination agent derived from an acid and being capable of releasing HCl *in situ* during chlorination is thionyl chloride.

The reaction of the compound (I), preferably the compound (Ia), comprised, preferably dissolved in the solvent system , and the chlorination agent, preferably thionyl chloride, generally can be conducted in any suitable way. Preferably, the chlorination agent, preferably thionyl chloride, is added to the solvent system comprising the compound (I), preferably the compound (Ia). The chlorination agent, preferably thionyl chloride, can be added either neat and/or as comprised in the solvent system. Preferably, it is added neat. The addition is accomplished by any suitable method, for example via a cannula and a syringe.

During addition of the chlorination agent, preferably thionyl chloride, to the solvent system comprising the compound (I), preferably the compound (Ia), according to step (2), the temperature of the solvent system is preferably kept below a given maximum, preferably below +20 °C, more preferably below +15 °C. Preferably, during addition of the chlorination agent, preferably thionyl chloride, according to step (2), the temperature of the solvent system is kept at a temperature in the range of from -20 to +10 °C, more preferably from -5 to +5 °C.

The amount of chlorination agent, preferably thionyl chloride, which is employed in step (2) generally depends on the amount of compound (I), preferably compound (Ia) being comprised in the solvent system. Preferably, 1.00 to 1.50 equivalents, preferably 1.05 to 1.30 equivalents, more preferably 1.10 to 1.25 equivalents, in each case with respect to the molar amount of compound of formula (I), preferably of compound of formula (Ia), of the chlorination agent, preferably thionyl chloride, are added.

After addition of the complete amount of chlorination agent, preferably thionyl chloride, the obtained reaction mixture is preferably stirred for a certain period of time to complete the reaction. Completeness of the reaction can be detected by known methods, for example thin layer chromatography, gas chromatography or preferably HPLC. Preferably, the reaction mixture is stirred for 0.5 to 2 hours, more preferably for 0.75 to 1.5 hours, such as for 1 hour. During stirring, the reaction mixture is preferably heated, preferably to a temperature of from +30 to +120 °C, more preferably from +30 to +110 °C. More preferably, the reaction mixture is heated to the boiling point of the solvent system. In particular in case a solvent system comprising THF and optionally containing at least one further ether is used, the reaction mixture is heated to the boiling point of the ether compound having the lowest boiling point. In particular in case a solvent system consisting of THF is used, the reaction mixture is heated to the boiling point of THF, i.e. to a temperature in the range of about +65 to about +67 °C at ambient pressure. More preferably, the heating of the reaction mixture is carried out under reflux conditions.

From the reaction and, thus, from step (2), the solvent system is obtained containing the compound according to general formula (IV) 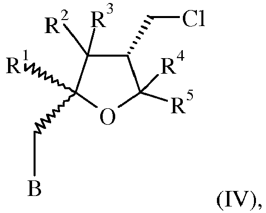 preferably formula (IVa) 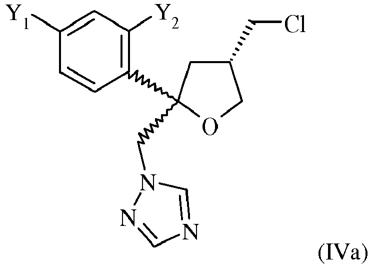 said compound containing the *cis*-isomer of general formula (V) 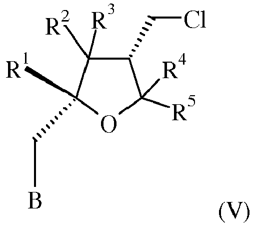 preferably formula (Va) 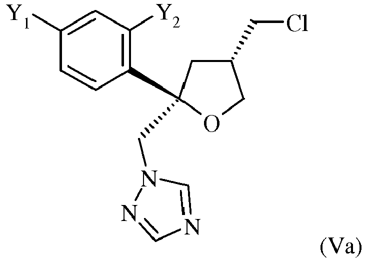 and/or its corresponding HCl salt, and the *trans-isomer* of general formula (VI) 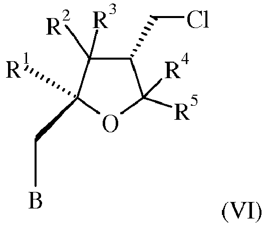 preferably (VIa) 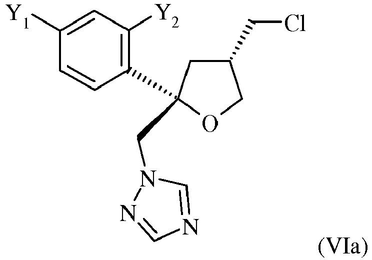 and/or its corresponding HCl salt. HCl which is present in the salts that are obtained from step (2) of the process of the present invention is generated *in situ* and has not to be added.

After the reaction of step (2) is completed, heating is most preferably stopped. In a subsequent step (3), the HCl salt of the compound of formula (IV), preferably (IVa), is preferably crystallised from the mixture obtained in (2). Preferably, the respectively obtained suspension contains a solid form of the HCl salt of compound of formula (IV), preferably (IVa), containing at least 97 %, preferably at least 98 % and more preferably at least 99 % of the HCl salt of the *cis*-isomer of formula (V), preferably (Va), and at most 3 %, preferably at most 2 % and more preferably at most 1 % of the HCl salt of the *trans-*isomer of formula (VI), preferably (VIa).

### Step (3)

It has surprisingly been found that the desired HCl salt of the *cis*-isomer of formula (V), preferably (Va), shows a significantly lower solubility in the solvent system according to the present invention, i.e. in the weakly complexing solvent system, at temperatures below reflux temperature of said solvent system, preferably below +40 °C, than the respective HCl salt of the *trans*-isomer of formula (VI), preferably (VIa). Therefore, from the crystallization in step (3), a suspension is most preferably obtained which contains, as solid material, the HCl salt of the *cis*-isomer of formula (V), preferably (Va), whereas the respective HCl salt of the *trans*-isomer of formula (VI), preferably (VIa)stays in solution. Optionally, minor amounts of the HCl salt of the *cis*-isomer of formula (V), preferably (Va) may be present in solution after crystallization. Further, the solid may contain very low amounts of the HCl salt of the *trans*-isomer of formula (VI), preferably (VIa). As mentioned above, preferably at most 3 %, more preferably at most 2 %, more preferably at most 1 % of the crystallized compound of general formula (IV), preferably (IVa) is the HCl salt of the *trans*-isomer of formula (VI), preferably (VIa). Thus, employing the advantageous solvent system, in particular the weakly complexing solvent system according to the present invention as described above in detail, it is possible to separate the HCl salt of the *cis*-isomer of formula (V), preferably (Va), in a straightforward method from the respective HCl salt of the *trans*-isomer of formula (VI), preferably (VIa) by simply heating and subsequent crystallization, a method which can be ideally realized in particular in industrial-scale processes. Furthermore, the agents and solvents that are used in steps (1) to (3), preferably (4) as described hereinbelow and optionally (5) as described hereinbelow, are non-toxic and do not provide difficult and therefore time- and money-consuming specifications for handling.

As to the crystallization according to step (3), no specific restrictions exist. Most preferably, during the crystallization in step (3), the reaction mixture obtained from step (2) is cooled in one, two, or more steps to a temperature of +40 °C or less, preferably from -20 to +40 °C, more preferably from -10 to +30 °C, more preferably from -10 to +20 °C, more preferably from -10 to +10 °C, more preferably from -5 to +5 °C. The term "cooling in one step" as used in this context of the present invention relates to a cooling method wherein the hot reaction mixture is continuously cooled from the temperature after step (2) to the final lower temperature mentioned above. The term "cooling in two steps" as used in this context of the present invention relates to a cooling method wherein the hot reaction mixture is continuously cooled from the temperature after step (2) to a first lower temperature or temperature range, kept at this temperature or within this temperature range for a certain period of time, and wherein the thus cooled reaction mixture is then further continuously cooled to a second lower temperature which is the final lower temperature mentioned above. During cooling, the mixture is preferably stirred.

According to a preferred embodiment of the process according to the present invention, cooling in step (3) is carried out in two steps. In the first step, the reaction mixture obtained from step (2) is cooled to a temperature in the range of from +15 to +30 °C, more preferably from +20 to +25 °C. During cooling, a solid precipitates, and a suspension containing said solid is obtained. Preferably, in a second step, this cooled suspension is further cooled, preferably to a temperature in the range of from -20 to +10 °C, more preferably from -10 to +10 °C, more preferably from -5 to +5 °C.

According to a conceivable embodiment of the process according to the present invention, the reaction mixture which is obtained in step (2) can be treated with seed crystals of the desired HCl salt of the *cis*-isomer of formula (V), in particular the HCl salt of the *cis-*isomer of formula (Va). This treatment may be conducted at a temperature of the reaction mixture in the range of from +20 to +40 °C.

Crystallization according to step (3) is generally conducted for a time long enough to crystallize the major portion, preferably more than 50 % by weight, more preferably at least 55 % by weight, more preferably at least 60 % by weight, more preferably at least 65 % by weight of the HCl salt of the *cis*-isomer of formula (V), preferably (Va), contained in the reaction mixture obtained from step (2). As mentioned above, preferably at most 3 %, more preferably at most 2 %, more preferably at most 1 % of the thus crystallized solid is the HCl salt of the *trans*-isomer of formula (VI), preferably (VIa).

### Step (4)

The suspension obtained from step (3) is preferably subjected to a further step (4) wherein the solid form of the HCl salt of the compound of formula (IV), preferably (IVa), is separated from the suspension. Since the suspension obtained from step (3) may contain, apart from the solvent system and the *cis*-isomer and the *trans*-isomer, either as solid or dissolved, further compounds such as residual chlorination agent and/or remainders of the chlorination agents obtained from the chlorination reaction, it is further preferred to subject the separated solid to at least one washing stage. After the preferred washing, the solid can be subjected to at least one drying stage. If necessary, it is conceivable to subject the dried solid to further washing and subsequent further drying.

Separation according to step (4) can be accomplished by any method known to the skilled person, for example by filtration, centrifugation or decanting, with filtration being preferred. Generally, filtration can be conducted at atmospheric pressure or at a pressure reduced or elevated relative to atmospheric pressure. For example, filtration can be carried out at a pressure in the range of from 0.1 to 1.0 bar or from 1.1 to 5 bar. Further, separation such as filtration according to step (4) can be conducted under an inert atmosphere of, for example, nitrogen or argon, or in air or lean air, with air being preferred.

After separation of the desired HCl salt of the *cis*-isomer of formula (V), preferably (Va), the solid can be subjected to washing with at least one washing agent to remove impurities which may adhere to the solid, for example acid, remaining chlorination agent or residual HCl salt of the *trans*-isomer of formula (VI), preferably (VIa). Washing is preferably conducted with the same weakly complexing solvent as used in step (1), preferably tetrahydrofuran.

The solid obtained after separation and preferably washing can be subjected to at least one drying stage, for example to drying at elevated temperature and/or *in vacuo,* to at least partially, preferably essentially completely remove the solvent system and/or the washing agent, to obtain the desired HCl salt of the *cis*-isomer of formula (V), preferably (Va), in dry form. Drying *in vacuo* is preferred wherein the pressure is preferably 500 mbar at most, more preferably 100 mbar at most, more preferably 50 mbar at most, with the drying temperature being preferably in the range of from +30 to +55 °C, more preferably from +40 to +50 °C.

The desired HCl salt of the *cis*-isomer of formula (V), in particular formula (Va), that is obtained by the process according to the present invention is most preferably present in at least partially crystalline, particularly preferably crystalline form.

According to a conceivable embodiment of the present invention, the HCl salt of the compound of formula (IV), preferably (IVa), as obtained from step (3), preferably from step (4), may be transferred to the corresponding hydroxyl compound of formula (I), preferably (Ia), if desired, by a conceivable method known to the skilled person. Thus, compared to the compound of formula (I), preferably (Ia), as employed in step (1), i.e. a compound preferably comprising from 80 to 95 %, preferably from 85 to 95 % of the *cis-*isomer of formula (II), preferably (IIa), and from 20 to 5 %, preferably from 15 to 5 % of the *trans*-isomer of formula (III), preferably (IIIa), an essentially *cis*-pure compound of formula (IV), preferably (IVa), can be obtained, containing preferably at most 3 %, more preferably at most 2 %, more preferably at most 1 % of the *trans*-isomer of formula (III), preferably (IIIa).

The HCl salt of the compound according to formula (IV), preferably (IVa), as obtained from step (3), preferably step (4), may be directly used in a synthesis process of an antifungal agent, in particular of posaconazole. Therefore, the present invention relates to the use of the HCl salt of a preferably at least partially crystalline, particularly preferably crystalline, compound of formula (IV), preferably (IVa), containing at least 97 %, preferably at least 98 % and more preferably at least 99 % of the HCl salt of the *cis*-isomer of formula (V), preferably (Va), and at most 3 %, preferably at most 2 % and more preferably at most 1 % of the HCl salt of the *trans*-isomer of formula (VI), preferably (VIa), for the preparation of an antifungal agent or a pharmaceutically acceptable salt thereof, preferably, wherein both Y₁ and Y₂ are F, of a compound of formula (X) 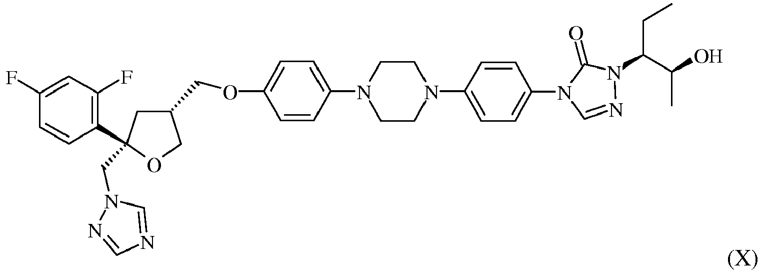 or a pharmaceutically acceptable salt thereof.

Further, the present invention relates to a process for the preparation of an antifungal agent or a pharmaceutically acceptable salt thereof, preferably of a compound of formula (X) 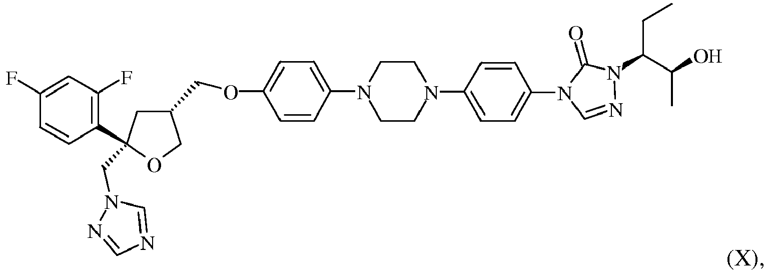 or a pharmaceutically acceptable salt thereof, wherein the HCl salt of a preferably at least partially crystalline, particularly preferably crystalline, compound of formula (IV), preferably (IVa), containing at least 97 %, preferably at least 98 % and more preferably at least 99 % of the HCl salt of the *cis*-isomer of formula (V), preferably (Va), and at most 3 %, preferably at most 2 % and more preferably at most 1 % of the HCl salt of the *trans-*isomer of formula (VI), preferably (VIa), preferably, wherein both Y₁ and Y₂ are F, is used as a starting material.

### Step (5)

Preferably, the HCl salt of the compound of formula (IV), preferably (IVa), obtained in (3), preferably in (4), is treated in a further step (5) with at least one base to obtain the compound of formula (IV), preferably (IVa), containing at least 97 %, preferably at least 98 % and more preferably at least 99 % of the *cis*-isomer of formula (V), preferably (Va), and at most 3 %, preferably at most 2 % and more preferably at most 1 % of the *trans-*isomer of formula (VI), preferably (VIa).

According to this step (5), HCl is removed to obtain the compound according to formula (IV), preferably (IVa), as free chloride.

The treating according to step (5) generally can be conducted according to any suitable method known by the skilled person. In one embodiment the *cis*-isomer of the compound according to formula (IV), in particular the *cis*-isomer of the compound of formula (IVa), is first dissolved in a suitable solvent, for example an organic solvent. Suitable organic solvents include, but are not restricted to, ethers, esters such as ethyl acetate, chlorinated solvents such as CH₂Cl₂, ketones such as acetone and MIBK, alcohols such as methanol, ethanol or 2-propanol, and mixtures of two or more thereof. The thus obtained solution is then treated with the at least one base. Generally, any suitable base can be used. Suitable bases include, but are not restriced to, organic bases such as amines like triethylamine, diisopropylethylamine, pyridine, morpholine, *N*-methylmorpholine or piperidine, and inorganic bases such as carbonates of, bicarbonates of or, preferably hydroxides of alkaline metals or alkaline earth metals, like KOH, NaOH, and LiOH, and mixtures of two or more thereof. In particular the inorganic bases are most preferably employed as aqueous solution. If according to a preferred embodiment, an inorganic base is used as aqueous solution, the base is added in an amount so that the pH of the mixture is preferably in the range of from 6 to 7.

The *cis*-isomer of the compound according to formula (IV), preferably (IVa), which is present in the solution after the treatment with the base can be separated from the solution by any method known to the skilled artisan.

In case an inorganic base is employed in step (5) as aqueous solution, the resulting organic and aqueous layers are suitably separated, wherein the compound according to formula (IV), preferably (IVa), is predominantly present in the organic layer. Known work-up like drying of the organic layer, for example using MgSO₄ or Na₂SO₄, and removal of the organic solvent, for example at elevated temperature and/or subatmospheric pressure, optionally followed by drying at a temperature of +30 to +60 °C, preferably from +40 to +50 °C, yields the desired compound according to formula (IV), preferably (IVa), of which preferably at least 97 %, more preferably at least 98 %, more preferably at least 99 % consist of the *cis*-isomer according to formula (V), preferably (Va), as free chloride.

As mentioned above, the at least partially crystalline, more preferably crystalline compound according to formula (IV), preferably (IVa), as obtained from step (5), may be used in a synthesis process of an antifungal agent, in particular of posaconazole. Therefore, the present invention relates to the use of an at least partially crystalline, particularly preferably crystalline, compound of formula (IV), preferably (IVa), containing at least 97 %, preferably at least 98 % and more preferably at least 99 % of the *cis*-isomer of formula (V), preferably (Va), and at most 3 %, preferably at most 2 % and more preferably at most 1 % of the *trans*-isomer of formula (VI), preferably (VIa), for the preparation of an antifungal agent or a pharmaceutically acceptable salt thereof, preferably, wherein both Y₁ and Y₂ are F, of a compound of formula (X) 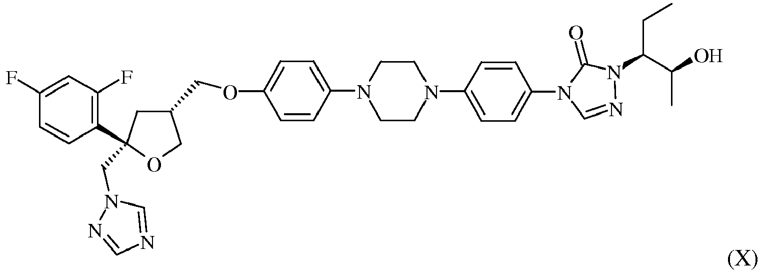 or a pharmaceutically acceptable salt thereof.

Further, the present invention relates to a process for the preparation of an antifungal agent or a pharmaceutically acceptable salt thereof, preferably of a compound of formula (X) 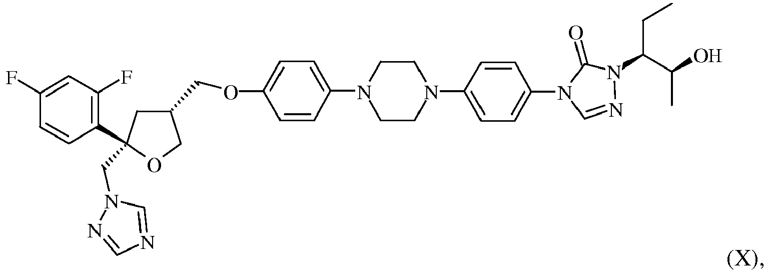 or a pharmaceutically acceptable salt thereof, wherein a preferably at least partially crystalline, particularly preferably crystalline, compound of formula (XIa) 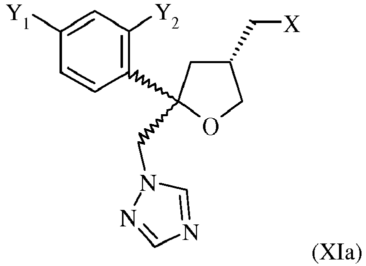 or its corresponding HX salt, wherein Y₁ and Y₂ are independently F or Cl, preferably F, and X is Cl, Br or I, preferably Cl, containing at least 97 %, preferably at least 98 % and more preferably at least 99 % of the *cis*-isomer of formula (XIIa) 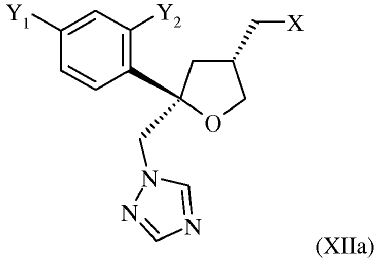 or its corresponding HX salt and at most 3 %, preferably at most 2 % and more preferably at most 1 % of the trans-isomer of formula (XIIIa) 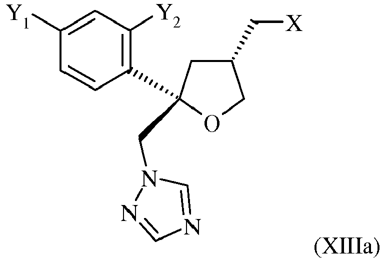 or its corresponding HX salt, is used as a starting material.

In particular, the present invention relates to said uses, or said processes, comprising reacting the *cis*-isomer of formula (Va) or its corresponding HCl salt with a compound of formula (VII) 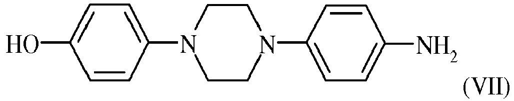 to give a compound of formula (VIII) 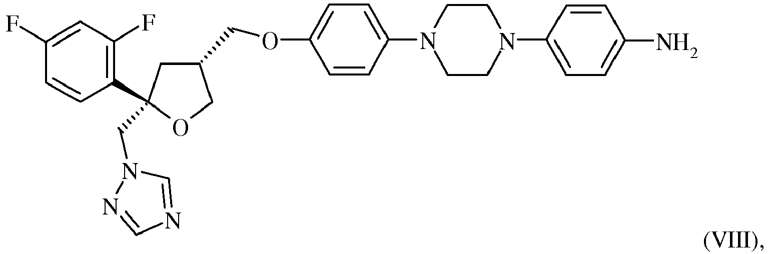 and suitably reacting the compound of formula (VIII) with a compound of formula (IX) 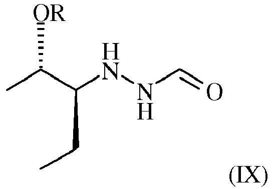 to give the compound of formula (X), wherein the residue R in formula (IX) is H or a suitable hydroxyl protecting group preferably selected from the group consisting of -SiRₐₐR_{bb}R_{cc} and optionally substituted alkyl, aryl, alkaryl or aralkyl residues, where Rₐₐ, R_{bb} and R_{cc} are the same or different and selected from the group consisting of optionally suitably substituted alkyl and aryl residues, R preferably being H. A preferred process for the preparation of the compound of formula (IX), with R = H, is described in WO 2011/144655 A1, as Step (6) on page 23, line 11 to page 25, line 29. There, the compound is referred to as compound of formula (V). Specific reference is made to Example 3 of WO 2011/144655 A1, from page 36, line 10 to page 37, line 19. A preferred process for the preparation of the compound of formula (IX), with R = -SiRₐₐR_{bb}R_{cc}, is described in WO 2011/144655 A1, as Step (7) on page 29, line 7 to page 30, line 2. There, the compound is referred to as compound of formula (VI).

### Step (6)

Further according to the present invention, the process may contain an additional step (6) according to which the compound of formula (IV), preferably (IVa), as obtained from step (5) and containing at least 97 %, preferably at least 98 % and more preferably at least 99 % of the *cis*-isomer of formula (V), preferably (Va) and at most 3 %, preferably at most 2 % and more preferably at most 1 % of the trans-isomer of formula (VI), preferably (VIa), is subjected to a halogen substitution reaction. Generally, the compound of formula (IV), preferably (IVa), is subjected to a step (6) wherein Cl is substituted by Br, or wherein Cl is substituted by I. Thus, the present invention provides a process according to which, starting from the compound (I), preferably (Ia), a compound (XI), preferably (XIa), with X = Br or I can be obtained of which at least 97 %, preferably at least 98 % and more preferably at least 99 % are present as *cis*-isomer of formula (XII), preferably (XIIa). Via this route, the present invention allows for an economically advantageous process for producing a highly *cis*-pure compound (XI), preferably (XIa), with X = Br or I, starting from the compound (I), preferably (Ia), comprised of from 80 to 95 %, preferably from 85 to 95 % such as from 89 to 91 %, of the *cis*-isomer, and of from 20 to 5 %, preferably from 15 to 5 % such as from 11 to 9 %, of the *trans*-isomer.

Therefore, the present invention also relates to the process comprising step (5) as defined above, further comprising
(6) treating the compound of formula (IV) obtained in (5), containing at least 97 %, preferably at least 98 % and more preferably at least 99 % of the *cis*-isomer of formula (V) and at most 3 %, preferably at most 2 % and more preferably at most 1 % of the *trans*-isomer of formula (VI), with at least one bromination agent or at least one iodination agent to obtain a compound of formula (XI) 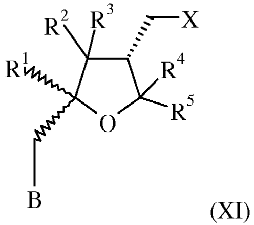 or its corresponding HX salt wherein X is Br or I, said compound of formula (XI) or its corresponding HX salt containing at least 97 %, preferably at least 98 % and more preferably at least 99 % of the *cis*-isomer of formula (XII) 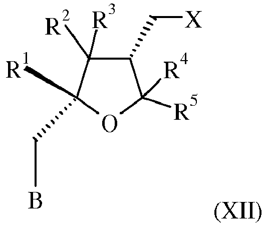 or its corresponding HX salt and at most 3 %, preferably at most 2 % and more preferably at most 1 % of the trans-isomer of formula (XIII) 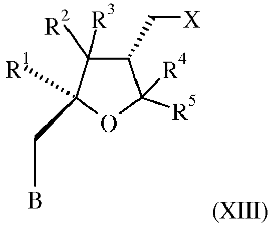
or its corresponding HX salt.

Concerning the halogen substitution reaction according to step (6), no specific restrictions exist provided the desired compounds are obtained.

According to a first embodiment, in case Cl is to be substituted by I, it is preferred to dissolve the compound obtained from step (5) in a preferably organic solvent, for example methyl-*iso*-butyl-ketone (MIBK). The iodination agent that is used to substitute the Cl atom can be selected from the known iodination agents, for example alkali metal iodides and earth alkaline metal iodides, preferably sodium iodide (NaI) and potassium iodide (KI) or mixtures thereof. Admixing the compound (IV), preferably (IVa) within the solvent with the iodination agent can be carried out under an inert atmosphere, for example under an atmosphere of nitrogen or argon, or in air or lean air, with air being preferred, the temperature being in the range of from +10 to +40 °C, more preferably from +20 to +30 °C. The concentration of the reaction mixture with regard to the compound of formula (IV), preferably (IVa), may vary between 0.005 and 0.05 mol/l, and is preferably in the range of from 0.01 to 0.05 mol/l. Treating the compound of formula (IV), preferably (IVa), in step (6) with the iodination agent is preferably carried out at elevated temperatures, preferably under reflux conditions. In the course of the treatment, it is preferred to suitably stir the reaction mixture. After the desired conversion, preferably after completion of the iodination reaction, heating is stopped and the resulting reaction mixture is cooled in one or more steps to a temperature of at most +30 °C, preferably at most +20 °C. The work-up of the obtained cooled reaction mixture can be conducted according to methods known by the skilled person. Preferably, the resulting reaction mixture is suitably concentrated, for example by evaporation under reduced pressure, and partitioning the resulting concentrate in an aqueous layer and an organic phase, wherein any suitable organic solvent such as DCM can be employed. The organic layer is preferably dried with at least one suitable drying agent such as sodium sulfate or the like, followed by removal of the solvent, for example by evaporation, preferably under reduced pressure. The thus obtained product can be, if desired, subjected to at least one suitable purification stage, for example chromatographic methods such as flash chromatography. According to this embodiment, the compound of formula (XI), preferably (XIa) is obtained wherein X = I, said compound of formula (XI), preferably (XIa) containing the *cis*-isomer of formula (XII), preferably (XIIa) in an amount of at least 97 %, preferably at least 98 %, more preferably at least 99 %, and the *trans*-isomer of formula (XIII), preferably (XIIIa), in an amount of at most 3 %, preferably at most 2 %, more preferably at most 1 %.

According to a second embodiment, in case Cl is to be substituted by Br, it is preferred to carry out this substitution analogously to the iodination reaction described above in the context of the first embodiment.

Therefore, the present invention in particular relates to the preferably at least partially crystalline, particularly preferably crystalline, compound of formula (XI) or its corresponding HX salt, obtainable or obtained by a process as described hereinabove, preferably by a process comprising, optionally consisting of, steps (1) and (2), or steps (1) to (3), or steps (1) to (4), or steps (1) to (5), or steps (1) to (6).

Also, the present invention relates to the preferably at least partially crystalline, particularly preferably crystalline, compound of formula (XI) or its corresponding HX salt containing at least 97 %, preferably at least 98 % and more preferably at least 99 % of the *cis*-isomer of formula (XII) or its corresponding HX salt and at most 3 %, preferably at most 2 % and more preferably at most 1 % of the *trans*-isomer of formula (XIII) or its corresponding HX salt.

Further, the present invention relates to the preferably at least partially crystalline, particularly preferably crystalline, compound of formula (XI) or its corresponding HX salt, wherein X is Cl, said compound being a compound of formula (IV) 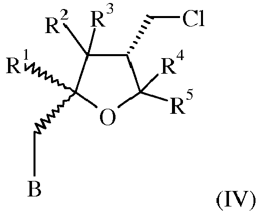 or its corresponding HCl salt, said compound of formula (IV) containing the *cis*-isomer of formula (V) 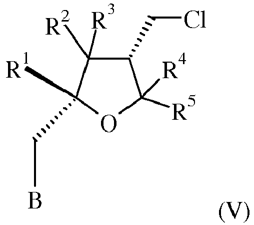 or its corresponding HCl salt, preferably containing at least 97 %, more preferably containing at least 98 %, more preferably containing at least 99 % of said *cis*-isomer of formula (V) or its corresponding HCl salt, and containing the *trans*-isomer of formula (VI) 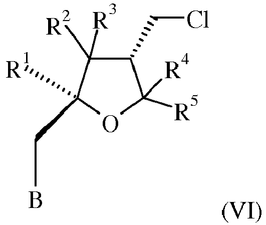 or its corresponding HCl salt, preferably containing at most 3 %, more preferably at most 2 %, more preferably at most 1 % of said *trans*-isomer of formula (VI) or its corresponding HCl salt. In formulas (IV), (V) and (VI) above, the residues R¹, R², R³, R⁴, R⁵ and B are as defined hereinabove.

In particular, the present invention relates to the compound of formula (XI), preferably (XIa), containing at least 97 %, preferably at least 98 %, more preferably at least 99 %, more preferably at least 99.9 %, of the *cis*-isomer of formula (XII), preferably (XIIa), and at most 3 %, preferably at most 2 %, more preferably at most 1 %, more preferably at most 0.1 %, of the *trans*-isomer of formula (XIII), preferably (XIIIa), wherein X is Cl, and Y₁ and Y₂ are preferably F.

Further particularly, the present invention relates to the compound of formula (XI), preferably (XIa), containing at least 97 %, preferably at least 98 %, more preferably at least 99 %, more preferably at least 99.9%, of the *cis*-isomer of formula (XII), preferably (XIIa), and at most 3 %, preferably at most 2 %, more preferably at most 1 %, more preferably at most 0.1 %, of the *trans*-isomer of formula (XIII), preferably (XIIIa), wherein wherein X is Br, and Y₁ and Y₂ are preferably F.

Further particularly, the present invention relates to the compound of formula (XI), preferably (XIa), containing at least 97 %, preferably at least 98 %, more preferably at least 99 %, more preferably at least 99.9 %, of the *cis*-isomer of formula (XII), preferably (XIIa), and at most 3 %, preferably at most 2 %, more preferably at most 1 %, more preferably at most 0.1 %, of the *trans*-isomer of formula (XIII), preferably (XIIIa), wherein X = I, and Y₁ and Y₂ are preferably F.

Further particularly, the present invention relates to the HX salt of the compound of formula (XI), preferably (XIa), containing at least 97 %, preferably at least 98 %, more preferably at least 99 %, more preferably at least 99.9 %, of the HX salt of the *cis*-isomer of formula (XII), preferably (XIIa), and at most 3 %, preferably at most 2 %, more preferably at most 1 %, more preferably at most 0.1 %, of the HX salt of the *trans*-isomer of formula (XIII), preferably (XIIIa), wherein X = Cl, and Y₁ and Y₂ are preferably F.

Further particularly, the present invention relates to the HX salt of the compound of formula (XI), preferably (XIa), containing 80 to 95 %, preferably from 85 to 95 % of the HX salt of the *cis*-isomer of formula (XII), preferably (XIIa), and 20 to 5 %, preferably from 15 to 5 % of the HX salt of the *trans*-isomer of formula (XIII), preferably (XIIIa), wherein X = Br, and Y₁ and Y₂ are preferably F.

The present invention is further illustrated by the following examples.

### Examples

### Example 1: Preparation of the compound of formula (Ia)

### (a) Preparation of the compound of formula (Ba)

In 20 ml of MTBE, 3.8 g of Mg were suspended. The temperature of the suspension was +55 °C. Then, 0.5 g of Grignard reagent (CH₃)₃Si-CH₂MgCl in MTBE from a previous batch were added in order to dry the system (if no such Grignard reagent is available for the first batch, (CH₃)₃Si-CH₂MgCl in diethyl ether (CAS Registry Number: 13170-43-9) commercially available as 1.0 M solution from Sigma-Aldrich can be used), followed by 1.0 ml of chloromethyl trimethyl silane (CM-TMS; CAS Registry Number: 2344-80-1; commercially available from Sigma-Aldrich). A solution of 14 ml of the CM-TMS in 43 ml of MTBE was added slowly over a period of 2 hours at a temperature of +55 °C. The mixture was stirred for 2 hours at +55 °C and then cooled to a temperature of -10 °C. Subsequently, 10.0 g of the commercial compound of formula (Aa) (CAS Registry Number: 51336-94-8; commercially available from Sigma-Aldrich) in 30 ml of MTBE were added and the temperature was kept in the range of from 0 to -10 °C. The reaction mixture was quenched in a 20 % (w/v) aqueous solution of ammonium chloride. The obtained organic layer was washed with a 20 % (w/v) aqueous solution of ammonium chloride. The thus washed organic layer was then washed with water.

To the organic layer, 11.0 ml of concentrated sulphuric acid were added, and the temperature was kept at +25 to +30 °C. Then, the reaction mixture was stirred at a temperature of from +45 to +50 °C for 3 hours. Subsequently, the reaction mixture was cooled to +20 °C and 25 ml of water were added, and the organic layer was separated. The obtained organic layer was extracted with a 9 % (w/v) aqueous solution of sodium bicarbonate, followed by washing with water. The solvents of the washed organic layer were removed by distillation under reduced pressure, and the compound of formula (Ba) was obtained as an oil.

The yield was 9.4 g, corresponding to a theoretical value of 95 %.

### (b) Preparation of the compound of formula (C) with Y₁ = Y₂ = F and R₁ = R₂ = CH₂CH₃

10.0 g of the compound of formula (Ba) (as oil, as obtained according to (a)) were dissolved in 20 ml of DMSO under stirring. Then, 3.2 g of NaOH flakes and 24.0 ml of diethyl malonate were added. The resulting suspension was stirred for 5 hours at +25 to +30 °C. Subsequently, 100 ml of water were added, and the resulting mixture was stirred for 30 min. The thus obtained solution was extracted with 80 ml of cyclohexane at +25 to +30 °C. After separation of the layers the aqueous layer was extracted with 40 ml of cyclohexane at +25 to +30 °C. The combined organic layers were washed with a 5% (w/v) aqueous solution of NaOH, followed by washing with water. After washing, the solvents of the organic layer were removed by distillation under reduced pressure and the title compound was obtained as an oil.

The yield was 15.0 g, corresponding to a theoretical value of 90.0 %.

### (c) Preparation of the compound of formula (D) with Y₁ = Y₂ = F

10.0 g of the compound of formula (C) as oil, as obtained according to (b), were dissolved in 120 ml of isopropyl alcohol and 13.0 ml of water under stirring at +25 to +30 °C. The resulting mixture was cooled to a temperature of from 0 to -5 °C. Then, 2.3 g of lithium chloride and 2.1 g of sodium borohydride were added at 0 to -5 °C. The resulting suspension was stirred at +25 to +30 °C for 20 hours. The pH of the stirred mixture was adjusted to a value of 1 (measured by using a calibrated pH meter) by addition of 4 N aqueous HCl. Afterwards, a 20 % (w/v) aqueous solution of NaOH was added to adjust the pH to a value of 10 (measured by using a calibrated pH meter). The resulting mixture was stirred for 1 hour. Then, the lower aqueous layer was drained. From the separated organic layer, the isopropyl alcohol was distilled off, and an oil was obtained. To the oil, 100 ml of toluene and 100 ml of water were added, and the product was extracted into the toluene layer. The solvents of the resulting toluene layer were removed by distillation, under reduced pressure and the compound of formula (D) with Y₁ = Y₂ = F was obtained as oil.

The yield was 6.0 g, corresponding to a theoretical value of 82.0 %.

### (d) Preparation of the compound of formula (E) with Y₁ = Y₂ = F

10.0 g of the compound of formula (D) as oil, as obtained according to (c), were dissolved in 80 ml of toluene and cooled to -15 °C. Then, 7.4 g of sodium bicarbonate, 0.5 g of enzyme (Novo SP 435, Candida Antarctica, Novozym 435 from Novo Nordisk), and 7.9 ml of isobutyric anhydride were added. The resulting mixture was stirred at -15 °C for 24 hours. Then the solids were filtered off and the filtrate was washed with a 5 % (w/v) aqueous solution of sodium bicarbonate, followed by washing with water. The solvents of the resulting organic layer were removed by distillation under reduced pressure to obtain the desired product as an oil. This oil was dissolved in 40 ml of n-heptane at +50 to +60 °C. The clear solution was gradually cooled to a temperature of +10 °C. The compound of formula (E) with Y₁ = Y₂ = F crystallized as colorless crystals. The obtained solids were filtered, and the wet filter cake was washed with 20 ml of n-heptane. The filter cake was then dried at +40 °C *in vacuo* and the compound of formula (E) with Y₁ = Y₂ = F was obtained as colorless crystals.

The yield was 9.2 g, corresponding to a theoretical value of 70.0 %.

### (e) Preparation of the compound of formula (F) with Y₁ = Y₂ = F and Hal = I

10.0 g of the crystals obtained in (d) were dissolved in 80 ml of ethyl acetate under stirring. The resulting solution was cooled to -15°C, and 21.5 g of iodine and 7.0 g of sodium bicarbonate were added. The obtained suspension was stirred at -15°C for 5 hours. The reaction mixture was quenched in 200 ml of a 10% (w/v) aqueous solution of sodium sulphite. The organic layer was washed with 100 ml of a 10% (w/v) aqueous solution of sodium sulphite, followed by washing with water. The solvents of the thus obtained, washed organic layer were removed by distillation under reduced pressure to obtain the title compound as an oil.

The yield was 13.5 g, corresponding to a theoretical value of 95.0%.

### (f) Preparation of the compound of formula (Ia) with Y₁ = Y₂ = F

10.0 g of the compound of formula (F) as oil, as obtained according to (e), were dissolved in 80 ml of DMSO under stirring. Then, 10 g of the sodium salt of 1,2,4-triazole were added at +25 to +30 °C, and the resulting reaction mixture was stirred for 24 hours at +85 to +90 °C. The mixture was then cooled to +25 to +30 °C, and 25 ml of a 5 % (w/v) aqueous solution of sodium hydroxide were added. The mixture was then stirred for 3 hours at +25 to +30 °C. 100 ml of water were added, and the product was extracted into 150 ml of methyl tetrahydrofuran. The thus obtained organic layer was washed with a 10 % (w/v) aqueous solution of sodium chloride, and subsequently the solvents of the resulting separated organic layer were removed by distillation under reduced pressure to obtain the compound of formula (Ia) with Y₁ = Y₂ = F as a crude oil.

The yield was 6.0 g, corresponding to a theoretical value of 86.0 %.

10.0 g of the crude oil were dissolved in 100 ml of methyl tetrahydrofuran under stirring at +50 to +60 °C. Then, 300 ml of n-heptane were added at +50 to +60 °C over a period of 30 min. The turbid solution was cooled to +25 to +30 °C and stirred for another 30 min. The resulting suspension was cooled to 0 to -5 °C and stirred for 2 hours. The product was filtered, and the wet cake was washed with 20 ml of n-heptane. The washed product was dried at +40 °C *in vacuo* to obtain the crystalline compound of formula (Ia) with Y₁ = Y₂ = F as a colorless solid.

The yield was 7.0 g, corresponding to a theoretical value of 70.0 %.

The compound of formula (Ia) with Y₁ = Y₂ = F was obtained as mixture of the *cis*-isomer with the respective *trans*-isomer with a *cis: trans* ratio of 9:1. HPLC method for the determination of purity and *cis*/*trans* ratio of compound of formula (Ia) with Y₁ = Y₂ = F:

| **Principle** | Determination by HPLC using UV detector | |
|---|---|---|
| **Reagents and Equipment** | Potassium dihydrogen phosphate | Merck Cat. No. 60487305001730 |
| | Orthophosphoric acid (85 %) | AR Grade e.g (Merck, Cat No 61768205001046) |
| | Acetonitrile | HPLC grade (e.g. Merck Cat. No. 61830025001046) |
| | HPLC system | Agilent 1100 series or similar |
| | pH meter | e.g. Metrohm or equivalent |
| **Buffer Preparation** | Dissolve 2.72 g of Potassium dihydrogen phosphate in 1000 ml of water and adjust the pH to 3.0 ± 0.05 by adding dilute orthophosphoric acid (85 %) using a pH meter. Filter through 0.45 µm (micrometer) filter and degas. | |
| **Diluent** | Buffer: Methanol (80: 20) v/v | |
| **Chromatographic Conditions** | | |
| Column | C₁₆, 250 mm X 4.6 mm i.d.5 µ, e.g. Ascentis RP amide or equivalent column can be used after appropriate validation. | |
| System | Gradient | |
| Column Temperature | +40 °C | |
| Mobile phase A | Buffer | |
| Mobile phase B | Buffer: Acetonitrile (30: 70) v/v | |
| Flow rate | 2.0 ml/min | |
| Injection temperature | +25 °C | |
| Injection volume | 25 µl (microliter) | |
| Run time | 45 minutes | |
| Detection wavelength | 210 nm | |
| System | Gradient | |

| | Time | % mobile phase B |
|---|---|---|
| | 0 | 20 |
| | 5 | 20 |
| | 15 | 40 |
| Gradient program | 25 | 80 |
| | 28 | 90 |
| | 39 | 90 |
| | 41 | 20 |
| | 45 | 20 |

### Example 2: Preparation of the HCl salt of the compound of formula (IVa) with Y₁=Y₂=F

A solution of 10.0 g of compound (Ia) (33.9 mmol, 1.0 equivalent) as obtained in Example 1 (f), in 100 mL tetrahydrofuran (THF) was treated with 3 mL of thionyl chloride (40.6 mmol, 1.2 equivalents), whilst the internal temperature was kept at 0 to +5 °C. Subsequently, the reaction mixture was heated to reflux for 60 min. After cooling to room temperature, a solid precipitated and the suspension was stirred for 60 min at 0 to +5 °C using an ice bath. The solid was separated via a G3-glass-frit and was subsequently washed with tetrahydrofuran (THF) (twice with 10mL in each case). After drying *in vacuo* (+45 °C, < 50 mbar) over night, the HCl salt of compound of formula (IVa) with Y₁ = Y₂ = F was obtained as white, crystalline solid. The yield was 7.75 g, 22.1 mmol, 65.3 % theoretical yield.

It was found that using HPLC analysis of the obtained product, the HCl salt of the compound of formula (IVa) contained no *trans-*isomer impurities and, thus, consisted of the HCl salt of the *cis*-isomer of formula (Va). Therefore, it is shown that the preferred process of the present invention allows for producing a 100 % *cis*-pure HCl salt of the compound of formula (IVa).

HPLC method for the determination of purity and *cis*/*trans* ratio of compound of formula (IVa) with Y₁ = Y₂ = F:

| | |
|---|---|
| HPLC-system: | Agilent series 1100/1200 |
| Column: | YMC - Pack Pro C 18, 3 micrometer, 4.6 x 150 mm HPLC column |
| Solvent System: | 50 % solvent A + 50 % solvent B, isocratic |
| Solvent A: | 40 mM Sulfaminic acid (7.77 g) + 1900 g H₂O + 7.8 g CH₃CN |
| Solvent B: | 40 mM Sulfaminic acid (7.77 g) + 500 g H₂O + 1176 g CH₃CN |
| Flow: | 1.0 mL/min |
| Stoptime: | 10.0 min |
| Posttime: | 0.5 min |
| Oven Temperature: | +40 °C |
| Wavelength: | 210 nm |
| Injection Volume: | 2.0 microliter |
| Sample Preparation: | Dissolve 10 mg of the compound in 5 mL CH₃CN, dilute with CH₃CN / water (1:1) to a total volume of 25 mL |

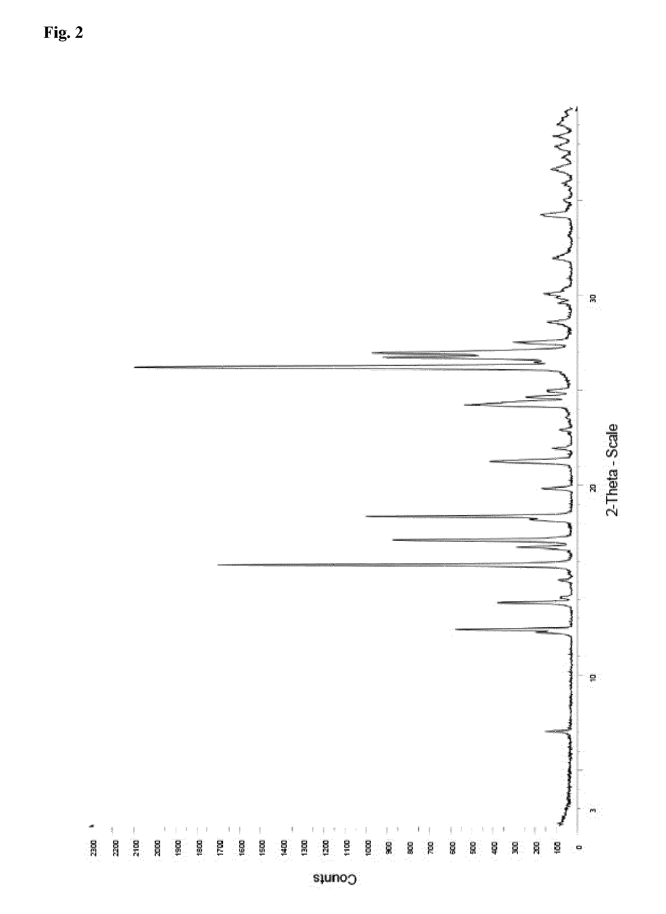

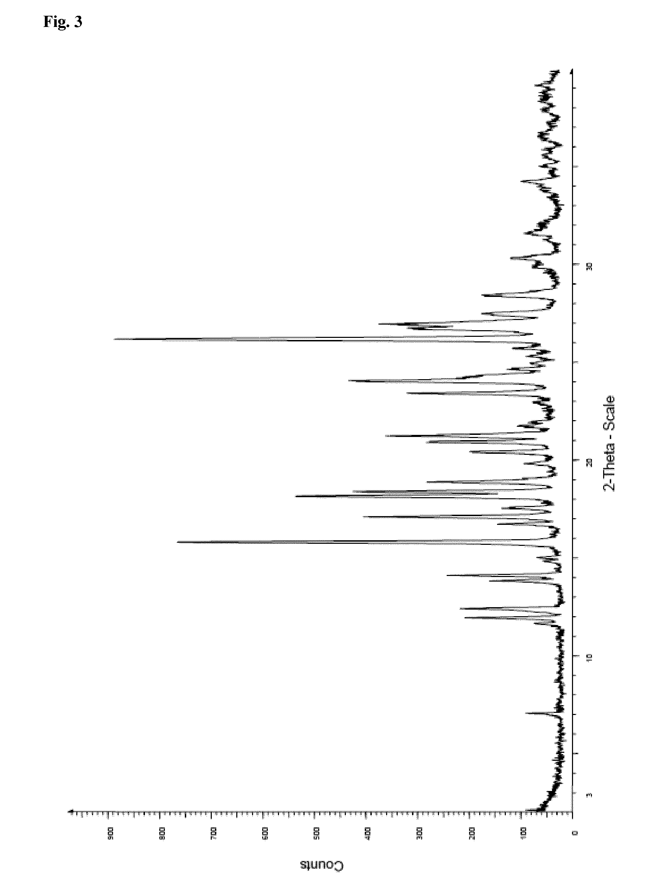

The X-ray powder diffraction pattern of the compound of formula (Va) as obtained (polymorphic form I) is shown in Figure 1. (Repeating the experiment using lower amounts of starting materials led also to polymorphic form II or a mixture of polymorphic forms I and II; the X-ray powder diffraction pattern of polymorphic form II is shown in Fig. 2, the X-ray powder diffraction pattern of said mixture of polymorphic forms I and II is shown in Fig. 3.)

The pattern regarding the polymorphic form I exhibits the following reflection list (the unit "Angstrom" is identical to 0.1 nm):

| angle / ° 2 Theta | d value / Angstrom | relative intensity / % |
|---|---|---|
| 11.63 | 7.610 | 26.8 |
| 11.93 | 7.418 | 40.3 |
| 14.12 | 6.272 | 32.7 |
| 14.82 | 5.976 | 6.0 |
| 15.69 | 5.649 | 13.6 |
| 17.55 | 5.054 | 23.9 |
| 18.16 | 4.884 | 99.0 |
| 18.87 | 4.702 | 49.9 |
| 20.42 | 4.350 | 67.6 |
| 20.91 | 4.248 | 53.8 |
| 21.74 | 4.089 | 14.0 |
| 23.43 | 3.793 | 100.0 |
| 23.49 | 3.794 | 82.2 |
| 24.06 | 3.696 | 97.3 |
| 25.36 | 3.509 | 8.1 |
| 25.74 | 3.459 | 13.0 |
| 26.27 | 3.390 | 10.5 |
| 27.43 | 3.249 | 23.2 |
| 28.46 | 3.133 | 32.4 |
| 29.37 | 3.039 | 3.3 |
| 30.38 | 2.940 | 15.5 |
| 31.24 | 2.861 | 9.0 |
| 31.63 | 2.826 | 17.3 |
| 32.23 | 2.775 | 3.5 |
| 33.39 | 2.681 | 4.5 |
| 33.79 | 2.651 | 8.4 |
| 34.04 | 2.632 | 8.0 |
| 35.54 | 2.524 | 7.6 |
| 36.40 | 2.466 | 6.7 |
| 37.03 | 2.426 | 4.7 |
| 38.23 | 2.352 | 4.4 |
| 38.80 | 2.319 | 7.6 |
| 39.42 | 2.290 | 2.7 |

The X-ray powder diffraction pattern was obtained with a X'Pert PRO diffractometer (PANalytical, Almelo, The Netherlands) equipped with a theta/theta coupled goniometer in transmission geometry, programmable XYZ stage with well plate holder, Cu-K_{alpha1,2} radiation source (wavelength 0.15419 nm) with a focussing mirror, a 0.5 ° divergence slit, a 0.02 ° soller slit collimator and a 0.5 ° anti-scattering slit on the incident beam side, a 2 mm anti-scattering slit, a 0.02 ° soller slit collimator, a Ni-filter and a solid state PIXcel detector on the diffracted beam side. The pattern was recorded with a tube voltage of 40 kV, tube current of 40 mA, a stepsize of 0.013 ° 2 Theta with 80 s per step in the angular range of 2 ° to 40 ° 2 Theta.

The ¹H-NMR spectrum of the compound of formula (Va), polymorphic form I, as obtained, is determined using a Bruker NMR apparatus, type AC 300, also showed the absence of the *trans*-isomer of formula (VIa):
**¹H-NMR (CDCl₃, 300 MHz):** delta = 2.00 - 2.15 (m, 1H), 2.44 - 2.65 (m, 2H), 3.46 - 3.58 (m, 2H), 3.84 - 3.95 (m, 1H), 4.04 - 4.14 (m, 1H), 4.76 - 4.94 (m, 2H), 6.63 - 6.83 (m, 2H), 7.03 - 7.13 (m, 1H), 8.19 (s, 1H), 10.25 (s, 1H) ppm.

The X-ray powder diffraction pattern of the polymorphic form II shows the following reflection list (20 most intensive peaks):

| angle / ° 2 Theta | d value / Angstrom | intensity / % |
|---|---|---|
| 26.22 | 3.396 | 100 |
| 15.80 | 5.604 | 81 |
| 18.36 | 4.827 | 47 |
| 26.97 | 3.303 | 45 |
| 26.75 | 3.330 | 43 |
| 17.10 | 5.180 | 41 |
| 12.40 | 7.133 | 27 |
| 24.25 | 3.668 | 24 |
| 21.24 | 4.180 | 19 |
| 13.82 | 6.404 | 17 |
| 27.53 | 3.237 | 13 |
| 16.73 | 5.294 | 13 |
| 24.64 | 3.610 | 10 |
| 18.19 | 4.873 | 9 |
| 12.24 | 7.227 | 8 |
| 19.83 | 4.474 | 7 |
| 34.25 | 2.616 | 6 |
| 30.09 | 2.968 | 6 |
| 7.02 | 12.578 | 6 |
| 28.59 | 3.119 | 5 |

The X-ray powder diffraction pattern of the mixture of the polymorphic forms I and II shows the following reflection list (20 most intensive peaks):

| angle / ° 2 Theta | d value / Angstrom | intensity / % |
|---|---|---|
| 26.21 | 3.398 | 100 |
| 15.81 | 5.601 | 87 |
| 18.16 | 4.882 | 59 |
| 18.39 | 4.821 | 46 |
| 24.06 | 3.696 | 44 |
| 17.11 | 5.178 | 44 |
| 26.97 | 3.303 | 39 |
| 21.25 | 4.178 | 38 |
| 23.42 | 3.795 | 33 |
| 26.74 | 3.331 | 32 |
| 18.89 | 4.695 | 29 |
| 20.94 | 4.239 | 28 |
| 14.10 | 6.275 | 26 |
| 11.93 | 7.416 | 22 |
| 12.38 | 7.142 | 22 |
| 20.42 | 4.345 | 19 |
| 24.33 | 3.656 | 17 |
| 28.45 | 3.135 | 17 |
| 13.82 | 6.402 | 16 |
| 27.50 | 3.241 | 16 |

### Example 3: Preparation of the compound of formula (IVa) with Y₁ = Y₂ = F

Compound (Ia) as herein described and preferably obtainable as described in Example 1 (f) (200 mg, 0.68 mmol) was emulsified in a mixture of hexane (0.5 mL) and DCM (2 mL). The mixture was cooled to 0 to +5 °C (ice-bath) and treated with thionyl chlorid (121 mg, 74 microL, 1.016 mmol, 1.5 equiv). The clear solution was heated to reflux temperature (about +55 °C) and was stirred for a period of 2 h to achieve complete conversion. After cooling to ambient temperature, ice-cold water (3 mL), saturated aqueous NaHCO₃ (3 mL) and diethylether (5 mL) were added to the reaction mixture. The layers were separated and the organic layer was washed with saturated aqueous NaHCO₃ (2 mL). The organic layer was dried with Na₂SO₄ and the solvent was removed under reduced pressure to give a colorless solid which was dried under reduced pressure (< 50 mbar) at +45 °C overnight. The title compound was obtained in a yield of 84 % (178 mg, 0.57 mmol).

The compound of formula (IVa) was obtained as mixture of the *cis*-isomer with the respective *trans*-isomer.

HPLC method for the determination of purity and *cis*/*trans* ratio of compound of formula (IVa) with Y₁ = Y₂ = F: as described in Example 2.

The ¹H-NMR spectrum of the compound of formula (IVa) as obtained, determined using a Bruker NMR apparatus, type AC 300, showed the following characteristics:
**¹H-NMR (CDCl₃, 300 MHz):** delta = 1.93 - 2.05 (m, 1H, *cis* + *trans),* 2.07- 2.19 (m, 0.17H, *trans),* 2.45 - 2.59 (m, 1.6H, *cis),* 2.69 - 2.79 (m, 0.16H, *trans),* 3.19 - 3.38 (m, 2H, *cis* + *trans),* 3.57 - 3.69 (m, 1H, *cis* + *trans),* 4.01 - 4.09 (m, 1H, *cis* + *trans),* 4.27 - 4.34 (d, 0.16H, *trans),* 4.42 - 4.49 (d, 0.80H, *cis),* 4.49 - 4.55 (d, 0.19H, *trans),* 4.55 - 4.62 (d, 0.82H, *cis),* 6.74 - 6.87 (m, 2H, *cis* + *trans),* 7.28 - 7.46 (m, 1H, *cis* + *trans),* 7.79 (s, 0.81H, *cis),* 7.82 (s, 0.15H, *trans),* 8.06 (s, 0.23H, *trans),* 8.07 (s, 0.77H, *cis)* ppm.

### Example 4: Preparation of the compound of formula (XIa) with Y₁ = Y₂ = F and X=Br

Compound (Ia) as herein described and preferably obtainable as described in Example 1 (f) (5.0 g, 16.93 mmol) was dissolved in 48 % aqueous HBr (17.0 mL) and treated with conc. sulfuric acid (450 microL, 831 mg, 0.5 equiv.). The mixture was heated to reflux temperature (about +122 °C) and stirred for a period of 6 h. Subsequently, the solution was cooled to 0 °C, and the pH was adjusted to 7.2 by dropwise addition of 5M aqueous NaOH (about 36 mL). The aqueous layer was extracted with DCM (3 x 100 mL) and the combined organic layers were dried with Na₂SO₄. The solvent was evaporated under reduced pressure and the resulting brown residue was purified by flash-chromatography on silica gel (eluent used was heptane : ethyl acetate = 1:1) to give the title compound as a solid in a yield of 76.0 % (4.61 g, 12.88 mmol).

The compound of formula (XIa) was obtained as mixture of the *cis*-isomer with the respective *trans*-isomer.

The ¹H-NMR spectrum of the compound of formula (XIa) as obtained, determined using a Bruker NMR apparatus, type AC 300, showed the following characteristics:
**¹H-NMR (CDCl₃, 300 MHz):** delta = 1.89 - 2.01 (m, 1H, *cis* + *trans),* 2.07 - 2.19 (m, 0.13H, *trans),* 2.42 - 2.59 (m, 1.66H, *cis),* 2.70 - 2.80 (m, 0.19H, *trans),* 3.03 - 3.23 (m, 2H, *cis* + *trans),* 3.52 - 3.63 (m, 1H, *cis* + *trans),* 4.02 - 4.09 (m, 1H, *cis* + *trans),* 4.25 - 4.32 (d, 0.19H, *trans),* 4.40 - 4.47 (d, 0.82H, *cis),* 4.47 - 4.53 (d, 0.2H, *trans),* 4.54 - 4.62 (d, 0.82H, *cis),* 6.74 - 6.86 (m, 2H, *cis* + *trans),* 7.27 - 7.45 (m, 1H, *cis* + *trans),* 7.77 (s, 0.77H, *cis),* 7.81 (s, 0.17H, *trans),* 8.04 (s, 0.19H, *trans),* 8.06 (s, 0.76H, *cis)* ppm.

### Example 5: Preparation of the compound of formula (IVa) with Y₁ = Y₂ = F from the corresponding HCl salt

The HCl salt of compound **(IVa)** as obtained in Example 2 (4.0 g, 11.4 mmol) was suspended in ethyl acetate (100 mL). The pH of the suspension was adjusted to 6.5 by addition of 2 % aqueous NaOH ( about 25 mL) to give a clear yellow biphasic mixture. The layers were separated and the organic layer was extracted with sat. aq. NaHCO₃ (80 mL). The combined organic layers were dried with Na₂SO₄ and the solvent was evaporated under reduced pressure. The residue was dried for a period of 60 min at +45 °C to give compound **(IVa)** with Y₁ = Y₂ = F as a colorless solid in a yield of 94.7 % (3.4 g, 10.8 mmol).

It was found that using HPLC analysis of the obtained product, the compound of formula **(IVa)** as obtained in this Example 5 contained no *trans*-isomer impurities and, thus, consisted of the *cis*-isomer of formula (Va).

HPLC method for the determination of purity and *cis*/*trans* ratio of compound of formula **(IVa)** with Y₁ = Y₂ = F: as described in Example 2.

The ¹H-NMR spectrum of the compound of formula (Va) as obtained, determined using a Bruker NMR apparatus, type AC 300, also showed the absence of the *trans*-isomer of formula (VIa):
**¹H-NMR (CDCl₃, 300 MHz):** delta = 1.91 - 2.03 (m, 1H), 2.40 - 2.57 (m, 2H), 3.18 - 3.36 (m, 2H), 3.59 - 3.67 (m, 1H), 3.99 - 4.07 (m, 1H), 4.41 - 4.61 (m, 2H), 6.73 - 6.85 (m, 2H), 7.26 - 7.36 (m, 1H), 7.77 (s, 1H), 8.05 (s, 1H) ppm.

### Example 6: Preparation of the compound of formula (XIa) with Y₁ = Y₂ = F and X=I

Compound (IVa) as obtained in Example 5 (0.5 g, 1.59 mmol) was dissolved in MIBK (75 mL). To this solution NaI (1.43 g, 9.45 mmol, 6 equiv.) was added. The mixture was heated to reflux temperature and stirred for a period of 48 h. The mixture was concentrated under reduced pressure and the resulting residue was partitioned between DCM (70 mL) and H₂O (70 mL). The layers were separated and the organic layer was dried with Na₂SO₄. The solvent was removed under reduced pressure to give the title compound as a brown oil in quantitative yield (0.65 g, 1.59 mmol).

### Example 7: Preparation of the compound of formula (X) from the HCl salt of the compound of formula (IVa) with Y₁ = Y₂ = F as starting material

To a solution of the compound of formula (VII), prepared according to the teaching of Example 1 of WO 01/34587 A2, page 6, line 1 to page 7, line 25, (149 mg, 0.554 mmol) in 925 microL of DMSO, solid NaOH (35.5 mg, 0.886 mmol, 1.6 equiv.) was added followed by solid NaI (83.0 mg, 0.554 mmol, 1.0 equiv.). The mixture is stirred for 10 min at +20 to +30 °C. Then, a solution of the compound of formula (Va) (200 mg, 0.638 mmol, 1.15 equiv.), prepared by treating the HCl salt of the compound **(IVa)** as obtained in Example 2 with aq. NaOH as described in Example 5 , in 925 microL of DMSO was added and the resulting brown mixture was allowed to stir for a period of 3 h at a temperature of +50 °C followed by a period of 3 h at +100 °C to achieve complete conversion (monitored by the HPLC method described below) of the compound of formula (Va). Subsequently, the mixture was cooled to +20 to +25 °C and water (2 mL) was slowly added to the reaction mixture. Next, the resulting solid was filtered off and washed with cold 1 % aq. NaOH (2 mL) followed by cold H₂O (2 x 2 mL). The crude product was purified by flash-chromatography on silica gel (eluent: DCM/ EtOH : 30/1 (v/v)), yielding 106 mg (0.194 mmol, 35%) of the compound of formula (VIII). The compound of formula (VIII) thus purified was then reacted to give Posaconazole according to the teaching of Examples 4 and 5 as described in WO 2011/144653 A1. The thus obtained Posaconazole was then treated according to the teaching of example 6 of WO 2011/144653 A1, wherefrom Posaconazole of polymorphic form IV was obtained.

### HPLC method:

| | |
|---|---|
| Instrument: | Agilent 1200 |
| Column: | Zorbax Eclipse XDB-C18, 4.6 x 150 mm, 3.5 micrometer |
| Eluent A: | 1700 mL 20 mmol phosphate buffer (pH 6.5) + 300 mL acetonitrile |
| Eluent B: | 1000 mL 20 mmol phosphate buffer (pH 6.5) + 1000 mL acetonitrile |
| Flow: | 1.0 mL/min |
| Temperature: | +40 °C |
| Injection volume: | 20 microL |
| Stop time: | 21 min |
| Wavelength: | 210 nm |
| Solvent for sample preparation: | CH₃CN / H₂O : 1/1 |

Sample preparation: Dissolve 100 microL of the reaction mixture in 20 mL of CH₃CN / H₂O : 1/1. Preparation of 2.0 L of 20 mmol phosphate buffer: Dissolve 2.11 g KH₂PO₄ and 4.27 g K₂HPO₄ in 1.8 L of H₂O, adjust the pH of this solution to 6.5 by adding 8.5 % H₃PO₄, adjust the total volume to 2.0 L by adding H₂O. Gradient:

| | | | | | |
|---|---|---|---|---|---|
| time [min] | 0 | 10 | 18 | 19 | 21 |
| eluent B [%] | 10 | 100 | 100 | 10 | 10 |

### Example 8: Preparation of the HX salt of the compound of formula (XIa) with Y₁ = Y₂ = F and X = Br

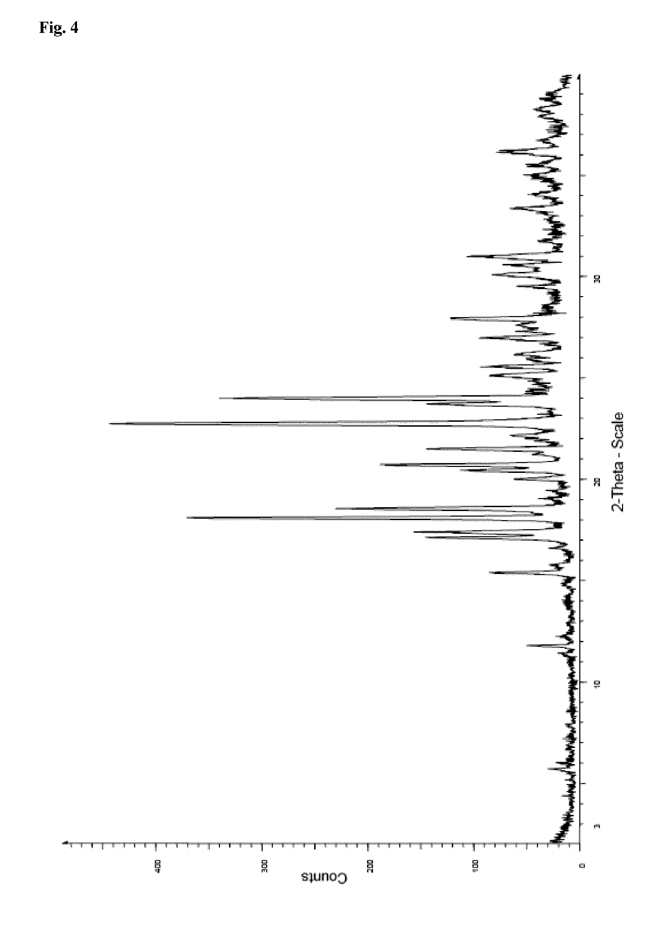

Thionyl bromide (430 mg, 160 microL, 2.07 mmol, 3.0 equiv.) was added to a solution of compound (Ia) as herein described and preferably obtainable as described in Example 1 (f), (204 mg, 0.69 mmol) in MTBE (7 mL) at a temperature of 0 to +5 °C. Then, the mixture was heated to reflux temperature and left to stir for a period of 6.5 h. Subsequently, the resulting suspension was cooled to 0 to +5 °C (ice-bath) and allowed to stir for 60 min. The solid was filtered off via a G3-sinter funnel and washed with MTBE (15 mL). After drying under vacuum at ambient temperature overnight, the title compound was obtained as a colorless solid in a yield of 96 % (291.8 mg, 0.66 mmol). The X-ray powder diffraction pattern of the compound of formula (XIa) as obtained is shown in Fig. 4. The pattern shows the following reflection list (20 most intensive peaks):

| angle / ° 2Theta | d value / Angstrom | intensity / % |
|---|---|---|
| 22.75 | 3.905 | 100 |
| 18.10 | 4.897 | 85 |
| 24.00 | 3.705 | 75 |
| 18.55 | 4.779 | 51 |
| 20.71 | 4.285 | 40 |
| 17.39 | 5.096 | 34 |
| 17.12 | 5.177 | 31 |
| 21.50 | 4.130 | 29 |
| 23.71 | 3.750 | 29 |
| 27.94 | 3.191 | 23 |
| 20.44 | 4.341 | 22 |
| 31.02 | 2.881 | 20 |
| 15.38 | 5.756 | 18 |
| 26.99 | 3.301 | 17 |
| 25.57 | 3.481 | 16 |
| 30.11 | 2.965 | 14 |
| 25.13 | 3.541 | 14 |
| 36.18 | 2.481 | 14 |
| 30.59 | 2.920 | 12 |
| 33.39 | 2.681 | 11 |

The compound of formula (XIa) was obtained as mixture of the *cis*-isomer with the respective *trans-*isomer.

The ¹H-NMR spectrum of the compound of formula (XIa) as obtained, determined using a Bruker NMR apparatus, type AC 300, showed the following characteristics:
**¹H-NMR (CDCl₃, 300 MHz):** delta = 2.06 - 2.37 (m, 1H, *cis* + *trans),* 2.39 - 3.04 (m, 2H, *cis* + *trans),* 3.26 - 3.85 (m, 2H, *cis* + *trans),* 3.93 - 4.13 (m, 1H, *cis* + *trans),* 4.15 - 4.33 (m, 1H, *cis* + *trans),* 4.74 - 5.22 (m, 2H, *cis* + *trans),* 6.71 - 7.00 (m, 2H, *cis* + *trans),* 7.10 - 7.35 (m, 1H, *cis* + *trans),* 8.33 (s, 0.82H, *cis),* 8.36 (s, 0.19H, *trans),* 10.28 (s, 0.81H, *cis),* 10.34 (s, 0.17H, *trans)* ppm.

### Cited Literature

- US 5,403,937
- EP 0 736 030 A1
- WO 95/17407
- WO 94/25452 A1
- WO 2011/144655 A1
- WO 01/34587 A2
- WO 2011/144653 A1
- WO 97/22710 A1

## Claims

1. A compound of formula (XI) 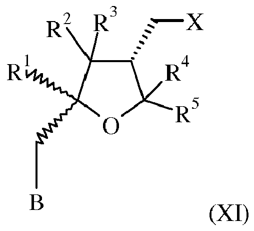 or its corresponding HX salt, wherein
R¹ is a linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂- aryl alkyl residue, or a substituted or unsubstituted C₅-C₂₂-heteroaryl residue;
R², R³, R⁴, R⁵ are, independently of each other, hydrogen, a linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂-aryl alkyl residue, a substituted or unsubstituted C₅-C₂₂-heteroaryl residue;
B is a basic substituent;
X is Cl, Br, or I;
said compound of formula (XI) or its corresponding HX salt containing the *cis-*isomer of formula (XII) 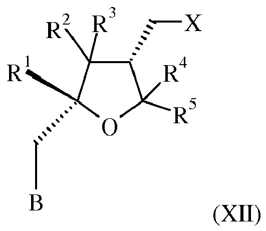 or its corresponding HX salt and the *trans*-isomer of formula (XIII) 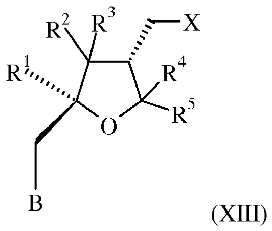 or its corresponding HX salt.

2. The compound of formula (XI) or its corresponding HX salt of claim 1, wherein R², R³, R⁴ and R⁵ are each hydrogen.

3. The compound of formula (XI) or its corresponding HX salt of claim 1 or 2, wherein R¹ is a 2,4-disubstituted phenyl, preferably of the following formula 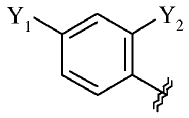 wherein Y₁ and Y₂ are independently F or Cl, preferably F.

4. The compound of formula (XI) or its corresponding HX salt of any of claims 1 to 3, wherein the basic substituent B is derived from a triazole compound of the following formula 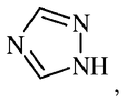 wherein B is preferably

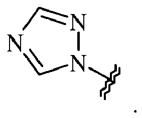

5. The compound of formula (XI) or its corresponding HX salt of any of claims 1 to 4, said compound of formula (XI) or its corresponding HX salt containing at least 97 %, preferably at least 98 % and more preferably at least 99 % of the *cis*-isomer of formula (XII) or its corresponding HX salt and at most 3 %, preferably at most 2 % and more preferably at most 1 % of the *trans-*isomer of formula (XIII) or its corresponding HX salt.

6. The compound of formula (XI) or its corresponding HX salt of any of claims 1 to 5, wherein X is Cl, said compound being a compound of formula (IV) 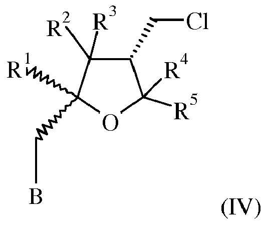 or its corresponding HCl salt, said compound of formula (IV) containing the *cis-*isomer of formula (V) 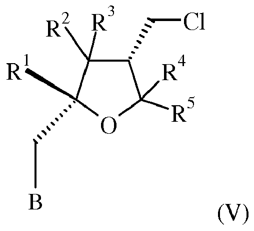 or its corresponding HCl salt, preferably containing at least 97 %, more preferably containing at least 98 %, more preferably containing at least 99 % of said *cis*-isomer of formula (V) or its corresponding HCl salt, and containing the *trans*-isomer of formula (VI) 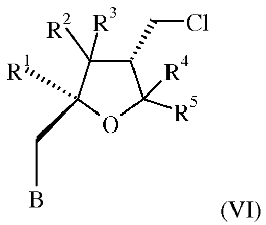 or its corresponding HCl salt, preferably containing at most 3 %, more preferably at most 2 %, more preferably at most 1 % of said *trans-*isomer of formula (VI) or its corresponding HCl salt.

7. The compound of formula (XI) or its corresponding HX salt of any of claims 1 to 6, being at least partially crystalline, preferably being crystalline.

8. A process comprising
(1) providing a compound of formula (I) 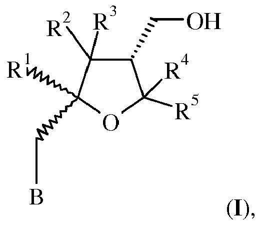 wherein
R¹ is a linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂- aryl alkyl residue, or a substituted or unsubstituted C₅-C₂₂-heteroaryl residue;
R², R³, R⁴, R⁵ are, independently of each other, a hydrogen, linear or branched, substituted or unsubstituted C₁-C₁₂-alkyl residue, a substituted or unsubstituted C₅-C₂₂-aryl residue, a linear or branched, substituted or unsubstituted C₆-C₂₂-alkyl aryl residue, a linear or branched, substituted or unsubstituted C₆-C₂₂-aryl alkyl residue, or a substituted or unsubstituted C₅-C₂₂-heteroaryl residue;
B is a basic substituent;
said compound of formula (I) containing the *cis*-isomer of formula (II) 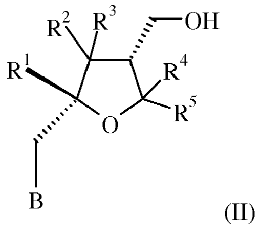 and the trans-isomer of formula (III) 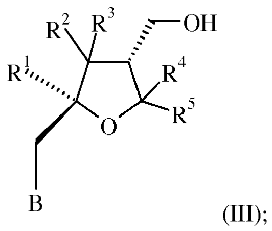
(2) treating the compound of formula (I) provided in (1) with at least one halogenation agent to obtain a compound of formula (XI) 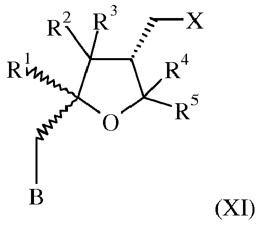 or its corresponding HX salt, containing the *cis*-isomer of formula (XII) 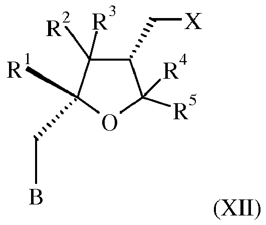 or its corresponding HX salt and the *trans*-isomer of formula (XIII) 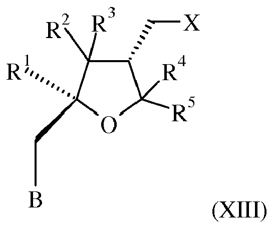 or its corresponding HX salt;
wherein X is Cl, Br, or I.

9. The process of claim 8, wherein R², R³, R⁴ and R⁵ are each hydrogen.

10. The process of claim 8 or 9, wherein R¹ is a 2,4-disubstituted phenyl, preferably of the following formula 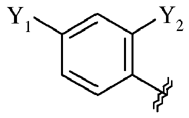 wherein Y₁ and Y₂ are independently F or Cl, preferably F.

11. The process of any of claims 8 to 10, wherein the basic substituent B is derived from a triazole compound of the following formula 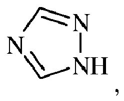 wherein B is preferably

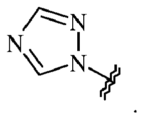

12. The process of any of claims 8 to 11, wherein the compound of formula (I) contains from 80 to 95 %, preferably from 85 to 95 % of the *cis*-isomer of formula (II) and from 20 to 5 %, preferably from 15 to 5 % of the *trans*-isomer of formula (III).

13. The process of any of claims 8 to 12, wherein X = Cl,
wherein in (1), the compound of formula (I) is provided comprised in a solvent system comprising tetrahydrofuran (THF), and
wherein in (2), the halogenation agent is a chlorination agent derived from an acid and is capable of releasing HCl *in situ* during chlorination,
from which process a mixture is obtained which contains the compound of formula (IV) 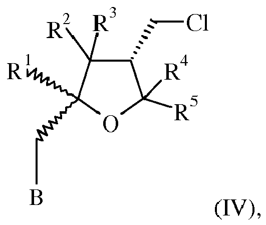 and/or its corresponding HCl salt, the compound of formula (IV) containing preferably at least 97 %, more preferably at least 98 %, more preferably at least 99 %, of the *cis*-isomer of formula (V) 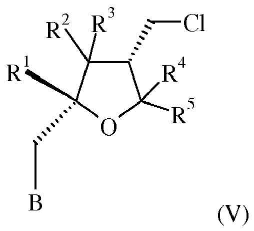 or its corresponding HCl salt and preferably at most 3 %, more preferably at most 2 %, more preferably at most 1 %, of the *trans*-isomer of formula (VI) 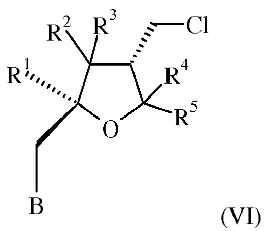 or its corresponding HCl salt.

14. The process of claim 13, wherein in (2), the chlorination agent derived from an acid and being capable of releasing HCl *in situ* during chlorination is thionyl chloride.

15. The process of claim 13 or 14, wherein in (2), 1.00 to 1.50 equivalents, preferably 1.05 to 1.30 equivalents, more preferably 1.10 to 1.25 equivalents, in each case with respect to the molar amount of compound of formula (I), of the chlorination agent are added.

16. The process of any of claims 13 to 15, wherein in (1), the solvent system comprising THF is THF as such or a mixture of THF and at least one further ether, said solvent system preferably consisting of THF.

17. The process of any of claims 13 to 16, wherein in (2), the treating comprises adding the chlorination agent to the compound to formula (I) comprised in the solvent system comprising THF at a temperature in the range of from -20 to +10 °C, preferably from -5 to +5 °C to obtain a mixture, and heating said mixture to a temperature in the range of from +30 to +120 °C, preferably from +30 to +110 °C.

18. The process of any of claims 13 to 17, said process further comprising
(3) crystallising the HCl salt of compound of formula (IV) from the mixture obtained in (2) to obtain a suspension containing a solid form of the HCl salt of the compound of formula (IV) containing at least 97 %, preferably at least 98 % and more preferably at least 99 % of the HCl salt of the *cis*-isomer of formula (V) and at most 3 %, preferably at most 2 % and more preferably at most 1 % of the HCl salt of the *trans*-isomer of formula (VI);
(4) preferably separating the solid form of the HCl salt of the compound of formula (IV) from the suspension obtained in (3).

19. The process of claim 18, wherein in (3), the crystallizing comprises cooling the mixture obtained in (2) to a temperature in the range of from -20 to +40 °C, preferably from -5 to +5 °C.

20. The process of claim 18 or 19, further comprising
(5) treating the HCl salt of the compound of formula (IV), preferably obtained in (4), with at least one base to obtain the compound of formula (IV) containing at least 97 %, preferably at least 98 % and more preferably at least 99 % of the *cis*-isomer of formula (V) and at most 3 %, preferably at most 2 % and more preferably at most 1 % of the *trans*-isomer of formula (VI).

21. The process of claim 20, further comprising
(6) treating the compound of formula (IV) obtained in (5), containing at least 97 %, preferably at least 98 % and more preferably at least 99 % of the *cis-*isomer of formula (V) and at most 3 %, preferably at most 2 % and more preferably at most 1 % of the *trans*-isomer of formula (VI), with at least one bromination agent or at least one iodination agent to obtain a compound of formula (XI) 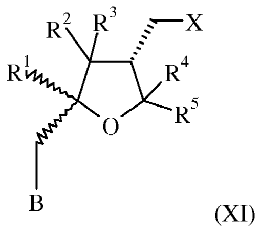 or its corresponding HX salt wherein X is Br or I, said compound of formula (XI) or its corresponding HX salt containing at least 97 %, preferably at least 98 % and more preferably at least 99 % of the *cis*-isomer of formula (XII) 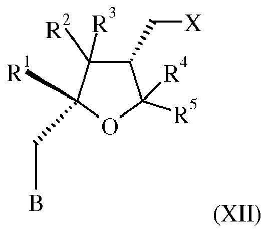 or its corresponding HX salt and at most 3 %, preferably at most 2 % and more preferably at most 1 % of the trans-isomer of formula (XIII) 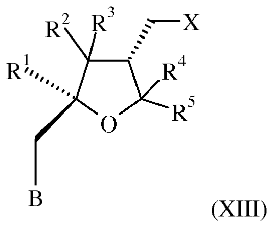 or its corresponding HX salt.

22. A compound of formula (XI) 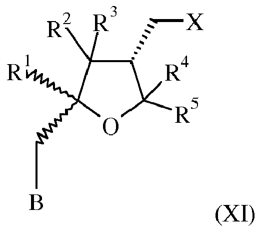 or its corresponding HX salt, obtainable or obtained by a process according to any of claims 8 to 21.

23. Use of a preferably at least partially crystalline, particularly preferably crystalline, compound of formula (XIa) 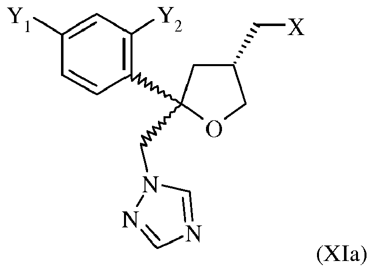 or its corresponding HX salt, wherein Y₁ and Y₂ are independently F or Cl, preferably F, and X is Cl, Br or I, preferably Cl, containing at least 97 %, preferably at least 98 % and more preferably at least 99 % of the *cis*-isomer of formula (XIIa) 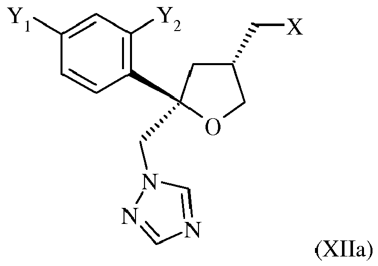 or its corresponding HX salt and at most 3 %, preferably at most 2 % and more preferably at most 1 % of the *trans*-isomer of formula (XIIIa) 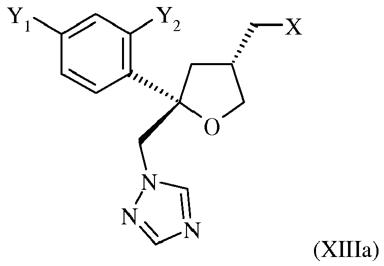 or its corresponding HX salt, for the preparation of an antifungal agent or a pharmaceutically acceptable salt thereof, preferably, wherein both Y₁ and Y₂ are F, of a compound of formula (X) 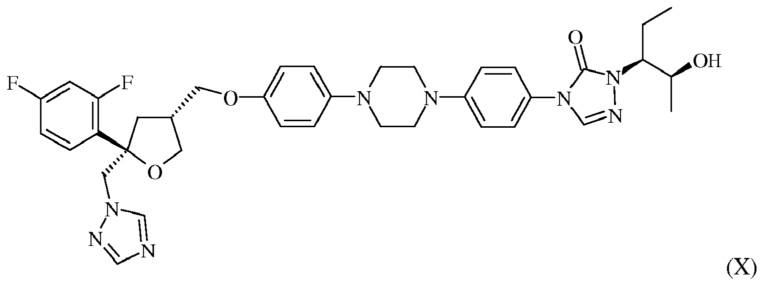 or a pharmaceutically acceptable salt thereof.

24. A process for the preparation of an antifungal agent or a pharmaceutically acceptable salt thereof, preferably of a compound of formula (X) 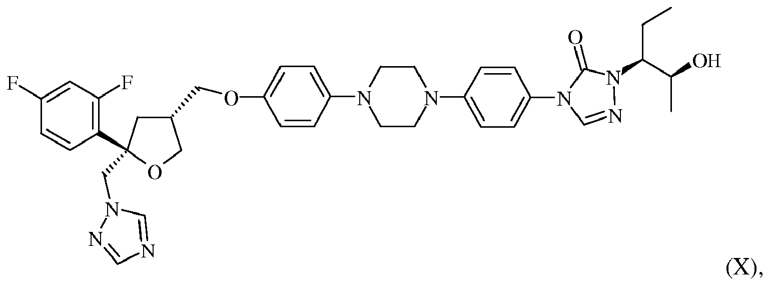 or a pharmaceutically acceptable salt thereof, wherein a preferably at least partially crystalline, particularly preferably crystalline, compound of formula (XIa) 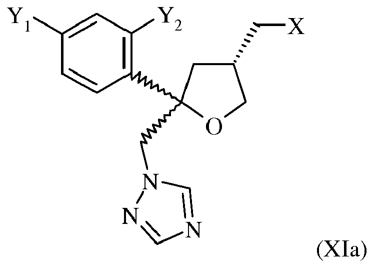 or its corresponding HX salt, wherein Y₁ and Y₂ are independently F or Cl, preferably F, and X is Cl, Br or I, preferably Cl, containing at least 97 %, preferably at least 98 % and more preferably at least 99 % of the *cis*-isomer of formula (XIIa) 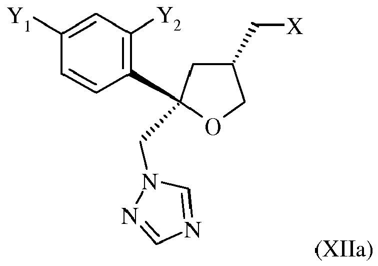 or its corresponding HX salt and at most 3 %, preferably at most 2 % and more preferably at most 1 % of the trans-isomer of formula (XIIIa) 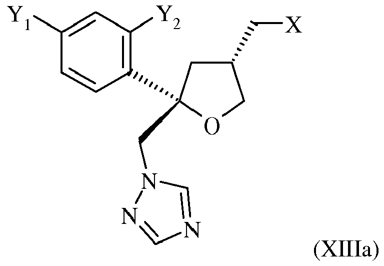 or its corresponding HX salt, is used as a starting material.
